# EUROPEAN PATENT SPECIFICATION

(11) **EP 1 506 039 B1**
(45) Date of publication and mention of the grant of the patent: **29.10.2008**
(21) Application number: 03725427.3
(22) Date of filing: 21.05.2003
(51) Int. Cl.: A61N 7/00, A61B 17/22, A61N 1/32

(54) **ABLATION DEVICE**
ABLATIONSVORRICHTUNG
INSTRUMENT POUR ABLATION

(30) Priority: 23.05.2002 GB 0211890; 23.05.2002 US 382833 P
(43) Date of publication of application: 16.02.2005
(73) Proprietor: Gendel Limited, Coleraine, County Londonderry BT52 1ST (GB)
(72) Inventor: MCHALE, A.P., Gendel Ltd., Science Innovation Ctr, Coleraine, Co. Londonderry BT52 1ST (GB); RUSSELL, L., Gendel Ltd.,Science Innovation Centre, Coleraine, Co. Londonderry BT52 1ST (GB)
(74) Representative: Harding, Charles Thomas
(86) International application number: PCT/GB2003/002201
(87) International publication number: WO 2003/099382

(56) References cited:
- WO-A-01/07011
- WO-A-02/40093
- US-A- 5 873 849
- US-A- 6 055 453
- US-A1- 2002 007 200
- US-A1- 2002 009 706

## Description

### FIELD

The present invention relates to a device capable of cell or tissue ablation; such a device may be used as a medical device for destroying tumours or other unwanted cells or tissues for example.

### BACKGROUND

In general therapeutic applications of ultrasound in the clinic may be divided into two major categories; applications that employ low intensity (0.125 - 3W/cm²) and those that employ higher intensities (≥ 5W/cm²) (ter Haar, (1999) Eur. J. Ultrasound 9:3). The former is commonly used in applications such as physiotherapy including the stimulation of normal physiological responses to injury or to accelerate some processes such as transport of drugs across the skin. Treatment with low intensity ultrasound rarely results in collateral tissue damage and indeed extreme efforts are employed to minimise such effects. This includes minimising excessive tissue heating as a result of exposure to the relevant dose of ultrasound. Usually this is accomplished by reducing the treatment time and/or delivering the ultrasound in a pulsed manner.

The major objective of applications involving the use of high intensity ultrasound is to selectively destroy tissue by hyperthermic processes. High intensity ultrasound-mediated tissue ablation may be further categorised on the basis in which the energy is delivered to the tissues. The ultrasound may be delivered directly from the transducer to the treatment area. Alternatively delivery may be mediated by a coupling device which results in focussing of the ultrasound. During the latter the ultrasound passing through intervening tissues is usually at low intensity and therefore relatively non-destructive. However at the focal point the accumulated energy is raised to a pre-determined higher intensity and tissue destruction occurs at or around that focal point. This has the advantage of being relatively selective and prevents major damage to intervening tissues.

In general the use of high intensity focussed ultrasound or HIFU exploits heating at the focal point and a number of methods together with devices for achieving focus and tissue ablation have been suggested (see US patents 4888746, 5895356, 5938608 and International Patent Applications WO 9735518A1 and WO 9922652A1).

In addition to a requirement for relatively sophisticated equipment to achieve focussing of high intensity ultrasound, one major disadvantage associated with the use of HIFU involves the potential for the occurrence of cavitation events which, in turn, leads to the formation of destructive or possibly mutagenic free radicals (Miller et al., (1996) Ultrasound in Med. & Biol. 22; 1131). An alternative approach involving a mechanism of sensitising the target tissue to low intensity ultrasound (either focussed or non-focussed) would therefore provide advantage.

It has been found that delivery of short, intense electric pulses to cell populations or tissues (*in vivo*) results in transient permeabilisation and this has provided the basis for what has become known as electrochemotherapy (Heller et al. Advanced Drug Delivery Rev. 35,119;1999). It was originally developed to facilitate passage of chemotherapeutic drugs into cancer cells which had become impermeable to those drugs. It has since been developed to a stage where delivery of electric pulses *in vivo* is being exploited in areas such as gene therapy in order to mediate introduction of DNA to target areas. Devices designed to facilitate delivery of the pulses *in vivo* under a variety of conditions (transdermal, laparoscopic, catheter, etc.) currently exist (International patent applications WO 9922809A1, WO 9906101A1 [Gentronics Inc.]; WO 9901157A1, WO 9901157A1, and WO 9901158 [Rhone Poulenc Rorer S.A.].

More recently it has been found that exposure of human erythrocytes to short and intense electric pulses which facilitates transient permeabilisation also results in a dramatic sensitisation to low intensity ultrasound (WO/01/07011).

### SUMMARY

The present invention relies on the demonstration that sensitisation of cells such as nucleated cells by application of an electric field ("electrosensitisation") renders the cells susceptible to ablation using low intensity ultrasound and thereby provides a means of eliminating unwanted tissues in the body. We therefore provide a device or apparatus capable of ablating a cell or tissue, by delivering an electric field and ultrasound to the cell or tissue.

According to a first aspect of the present invention, we provide an apparatus for ablation of nucleated cells, the apparatus comprising: (a) electric field signal generating circuitry for generating an electric field signal; (b) an electric field delivery component connected to receive the electric field signal and operable to deliver an electric field to a treatment site; (c) ultrasound signal generating circuitry for generating an ultrasound signal; (d) an ultrasound delivery component connected to receive the ultrasound signal and operable to deliver ultrasound to the treatment site; and (e) a controller operable to control (i) the electric field signal generating circuitry such that the electric field delivery component delivers an electric field of a strength operable to sensitise a nucleated cell at the treatment site, and (ii) the ultrasound signal generating circuitry in order to ablate a cell at the treatment site.

The invention also relies on the discovery that exposure of a cell to ultrasound followed by exposure to electric fields also results in cell disruption. Thus, exposure of a cell, preferably a nucleated cell to ultrasound and an electric field, applied in any order, results in cell disruption, and the apparatus as described here is capable of administering ultrasound and an electric field in either order to a cell,

However, in preferred embodiments, the electric field is generated and delivered by the apparatus to the cell in order to sensitised it, or electrosensitise it. Thus, preferably, the electric field is operable to sensitise a cell at the treatment site. Furthermore, in preferred embodiments, the ultrasound is generated and delivered by the apparatus to a sensitised cell (preferably an electrosensitised cell) in order to disrupt it Therefore, in such preferred embodiments of the invention, the ultrasound is operable to ablate a sensitised cell at the treatment site.

The electric field delivery component and the ultrasound delivery component may be housed in a common delivery head. Preferably, the controller is operable to provide the electric field before the ultrasound to the treatment site. Alternatively or in addition, the controller may be operable to provide the electric field and the ultrasound to the treatment site simultaneously.

The electric field delivery component may take a number of forms. It may for example comprise a single electrical contact. Alternatively, or in addition, the electric field delivery component may comprise a plurality of electrical contacts positioned at the vertices of a regular polygon, preferably a triangle, a square, a pentagon, a hexagon, or a heptagon. One or more further contact electrodes may be arranged within the circumference of the polygon. The electric field delivery component may comprise a plurality of contact electrodes arranged in a grid.

In a preferred embodiment, the or each of the contact electrodes comprises a needle. The needle may preferably be hollow, and/or electrically insulated along at least a portion of its extent, and may preferably comprise an extendible insulating sleeve.

A hollow needle is advantageous to deliver material to the vicinity of the cell being treated. Thus, in a preferred embodiment, the apparatus is operable to deliver a cell-death facilitating agent to the vicinity of the cell via the hollow needle.

The electric field delivery component may comprise an electrical contact comprising a conductive part of a contact pad. A plurality of electrical contacts may be coupled to a plurality of electric field signals in the apparatus. Preferably, the electric field signal generating circuitry is operable to deliver electric fields of between 1 V/cm and 10 kV/cm to the treatment site.

The apparatus may further advantageously comprise a diagnostic ultrasound component The ultrasound delivery component may comprise an ultrasound transducer. Where a plurality of ultrasound transducers are used, they may be coupled to a plurality of ultrasound signals. The ultrasound signal generating circuitry may be operable to deliver ultrasound at an average power density of between 0.1 W/cm² and 50 W/cm², preferably up to 7 W/cm², to the treatment site.

The ultrasound delivery component may be coupled to a tissue surface via a contact pressure vessel arranged to distort the tissue surface. Such an arrangement is advantageous in that it allows the ultrasound to be focussed and directed for example to a site internal to the patient being treated.

There is provided, according to a second aspect of the present invention, use of an apparatus according to the first aspect of the invention in a method of cell or tissue ablation *in vitro* or ex *vivo.*

We provide, according to a third aspect of the present invention, a method of ablating a cell *in vitro* or *ex vivo,* the method comprising the steps of: providing an apparatus according to the first aspects of the invention; generating an electric field signal to cause an electric field delivery component of the apparatus to deliver an electric field to a cell at a treatment site; and generating an ultrasound signal to cause an ultrasound delivery component of the apparatus to deliver an electric field to the cell.

Preferably, the apparatus comprises an electric field signal generating circuitry for generating an electric field signal and ultrasound signal generating circuitry for generating an ultrasound signal.

We further describe an electric field delivery component comprising one or more electrical contacts, in which the electrical contact or contacts comprise one or more hollow needles.

Preferably, the hollow needles are arranged in a grid or in which the hollow needles are arranged in a grid. Alternatively or in addition, the hollow needles are positioned at the vertices of a regular polygon, preferably a triangle, a square, a pentagon, a hexagon, or a heptagon. Optionally the electric field delivery component comprises one or more hollow needles arranged within the circumference of the polygon.

### BRIEF DESCRIPTION OF THE FIGURES

For a better understanding of the invention and to show how the same may be carried into effect reference is now made by way of example to the accompanying drawings in which:
Figure 1A is a schematic diagram of an embodiment of a tumour therapy device according to one aspect of the invention;
Figure 1B is a schematic diagram of another embodiment of a tumour therapy device according to one aspect of the invention;
Figure 2A is a schematic diagram of an electric field delivery component (EFD) for use in a tumour therapy device;
Figure 2B is a schematic diagram of the EFD shown in Figure 2A when employed in one orientation;
Figure 2C is a schematic diagram of the EFD shown in Figure 2A when employed in another orientation;
Figure 3A is a schematic perspective view of another EFD for use in a tumour therapy device;
Figure 3B is a schematic plan view of the EFD shown in Figure 3A;
Figure 4A is a schematic perspective view of another EFD for use in a tumour therapy device;
Figure 4B is a schematic plan view of the EFD shown in Figure 4A;
Figure 5A is a schematic perspective view of another EFD for use in a tumour therapy device;
Figure 5B is a schematic plan view of the EFD shown in Figure 5A;
Figure 6A is a schematic perspective view of another EFD for use in a tumour therapy device;
Figure 6B is a schematic plan view of the EFD shown in Figure 6A;
Figure 7A is a schematic side view of another EFD for use in a tumour therapy device;
Figure 7B is a schematic plan view of the EFD shown in Figure 7A;
Figure 8A is a schematic side view of a portion of an EFD for use in a tumour therapy device arranged to avoid electrical contact with essential tissue;
Figure 8B is a schematic side view of a portion of another EFD for use in a tumour therapy device arranged to avoid electrical contact with essential tissue;
Figure 9 is a schematic side view of a portion of an EFD for use in a tumour therapy device arranged to supply an electric field signal to surgically embedded contact electrodes;
Figure 10A is a schematic plan view of a portion of an EFD for use in a tumour therapy device comprising conductive parts of a contact pad;
Figure 10B is a schematic side view of the portion of the EFD shown in Figure 10A;
Figure 11A is a schematic plan view of a contact electrode configuration on a contact pad;
Figure 11B is a schematic plan view of another contact electrode configuration on a contact pad;
Figure 11C is a schematic plan view of another contact electrode configuration on a contact pad;
Figure 12 is a schematic side view of an EFD for use in a tumour therapy device including both needles and conductive parts of a contact pad as contact electrodes;
Figure 13A is a schematic side view of an ultrasound delivery component (USD) for use in a tumour therapy device coupled to a tissue mass by a mediating gel;
Figure 13B is a schematic side view of a USD for use in a tumour therapy device coupled to a tissue mass by an adhesive pad;
Figure 14 is a schematic side view of a USD for use in a tumour therapy device coupled to a tissue mass by a low pressure contact vessel to cause tissue distortion and facilitate ultrasound focussing;
Figure 15 is a schematic view of a lower portion of a USD for use in a tumour therapy device comprising three directional ultrasound transducers;
Figure 16 is a schematic view of a USD combined with a diagnostic ultrasound delivery and scanning component (DDS);
Figure 17 is a schematic view of a combined delivery head comprising an EFD and a USD in operation;
Figure 18 is a schematic view of a combined delivery head comprising an EFD, a USD and a DDS in operation.
Figures 19 to 33 show that a combination of electric field and ultrasound is capable of destroying a cell.

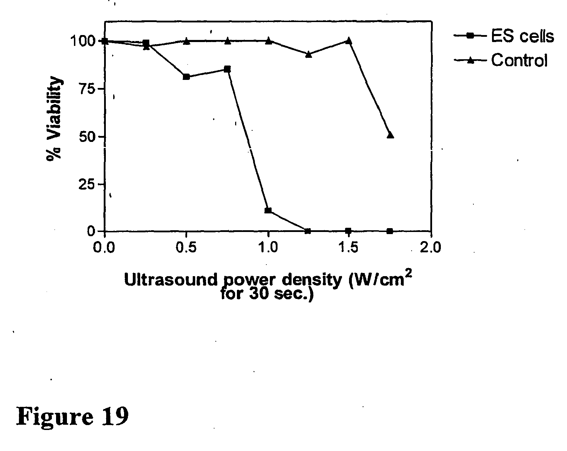

Figure 19 is a graph of the effect of ultrasound on control (▲) and electro-sensitised (■) 707 cells in suspension. Cells are electrosensitised by treatment with electric pulses of 3.625kV/cm at 1µF and cell viability is determined immediately following treatment with ultrasound.

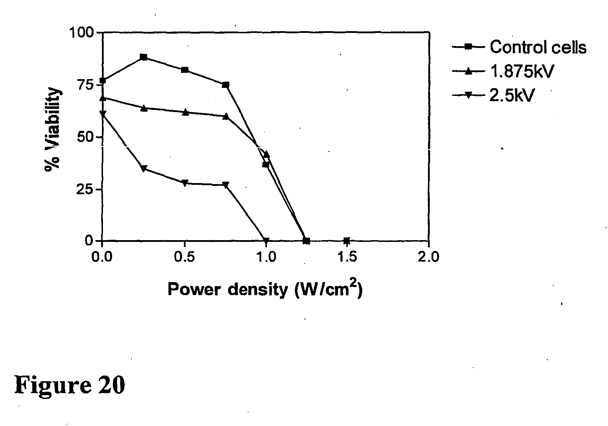

Figure 20 is a graph of the effect of ultrasound on control cells (■), cells electro-sensitised at 1.875kV/cm at 1µF (▲) and cells electro-sensitised at 2.5kV/cm (V). Cell viability is determined 1 hour after exposure to ultrasound.

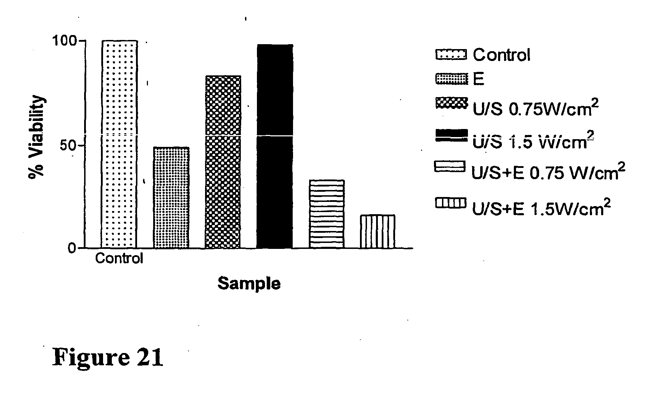

Figure 21 is a bar chart showing the effect of ultrasound on control and electro-sensitised cells immobilised in calcium alginate matrices.

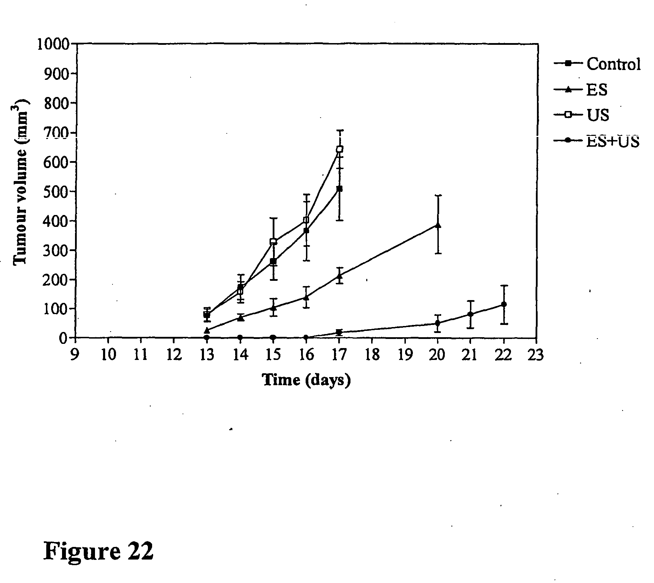

Figure 22 is a graph testing induction of tumours in C3H mice following treatment of a RIF-1 cell line with electric fields (▲), ultrasound (□) and electric fields in combination with ultrasound (●). Control populations (■) consist of cells which receive no treatment. The x-axis represents time measured in days and the y-axis represents tumour volume measured in mm³.

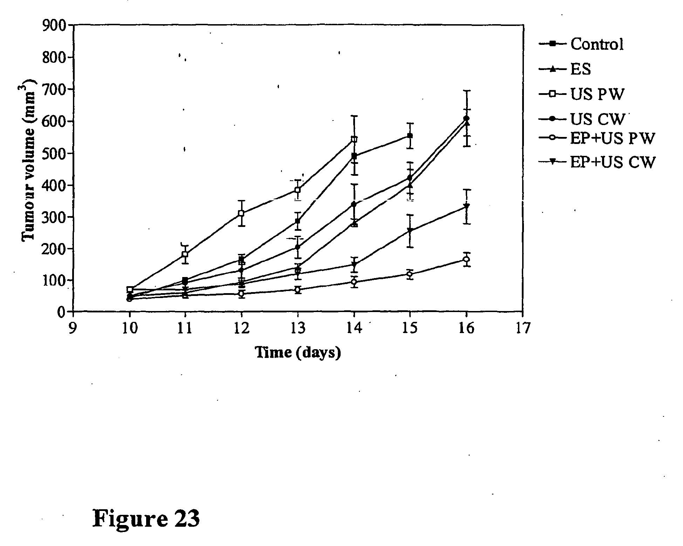

Figure 23 shows treatment of RIF-1 tumours *in situ* in mice with electric fields (▲), pulsed wave ultrasound (□), continuous wave ultrasound (●), electric field plus continuous wave ultrasound (V) and electric fields plus pulsed wave ultrasound (○). Continuous wave ultrasound is delivered at 0.7W/cm² at 3MHz for 2 minutes, while pulsed wave ultrasound is delivered at 1.8 to 1.9 W/cm² at 3MHz for 2 minutes at a 35% setting. Electric fields are delivered at 1.333 kV/cm. Control tumours (■) receive no treatment. The x-axis represents time measured in days and the y-axis represents tumour volume measured in mm³. Error bars represent +/- SEM (standard error mean).

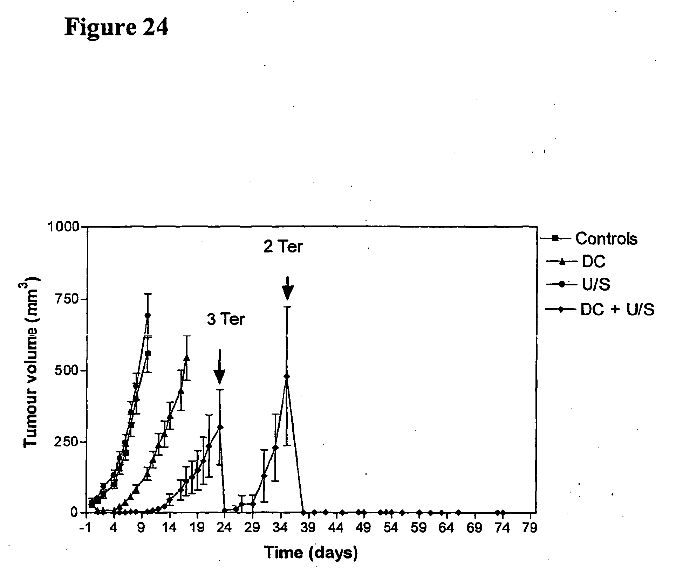

Figure 24 is a graph showing prolonged monitoring of experiments in which tumours were sensitised with direct current (DC) and subsequently treated with ultrasound. Groups of 6 animals are employed in each group and tumours are treated with ultrasound alone (●), DC alone (▲) and DC plus ultrasound (◆). Control animals (■) receive no treatment. The bold arrow insets together with 'Ter' indicates removal of animals from the experiment. Error bars represent +/- SEM (standard error mean).

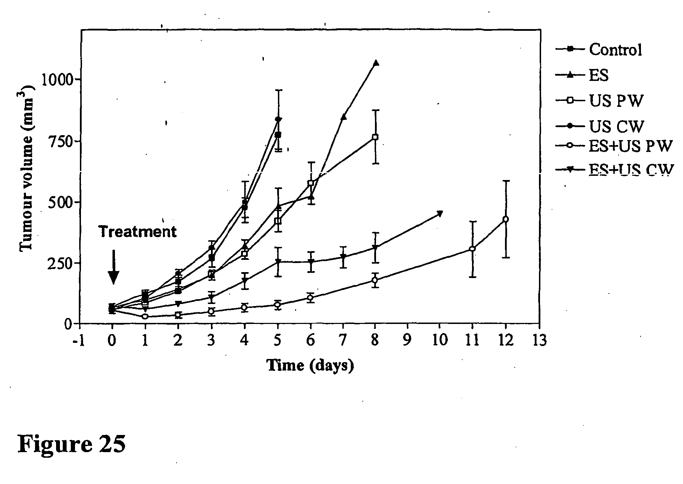

Figure 25 shows treatment of RIF-1 tumours *in situ* in mice with electric fields (▲), pulsed wave ultrasound (□), continuous wave ultrasound (●), electric field plus continuous wave ultrasound (V) and electric fields plus pulsed wave ultrasound (○). Continuous wave ultrasound is delivered at 1.25W/cm² at 3MHz for 2 minutes, while pulsed wave ultrasound is delivered at 2.5 W/cm² at 3MHz for 2 minutes at a 35% setting. Electric fields are delivered at 1.33 kV/cm. Control tumours (■) receive no treatment. The x-axis represents time measured in days and the y-axis represents tumour volume measured in mm³. Error bars represent +/- SEM (standard error mean).

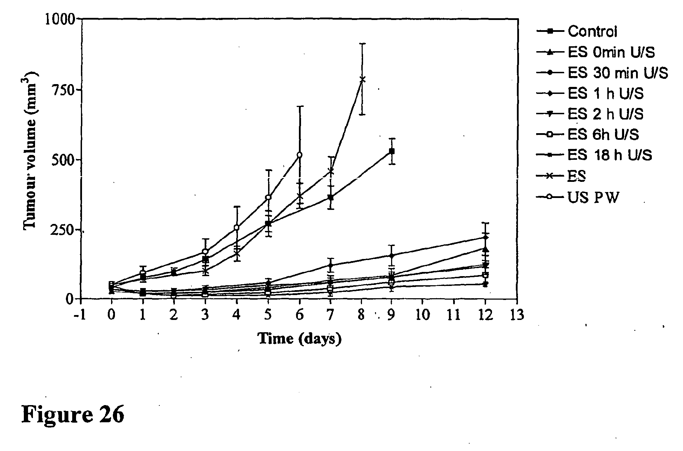

Figure 26 is a graph showing results of treatment of electrosensitised tumours with ultrasound at 0 (A), 0.5 (●), 1 (◆), 2 (V), 6 (□) and 18 (*) hours after electrosensitisation. Control populations consisted of untreated (■) or treated with electric pulses (×) or ultrasound (○) alone. In these experiments error bars represent ±SEM where n=3.

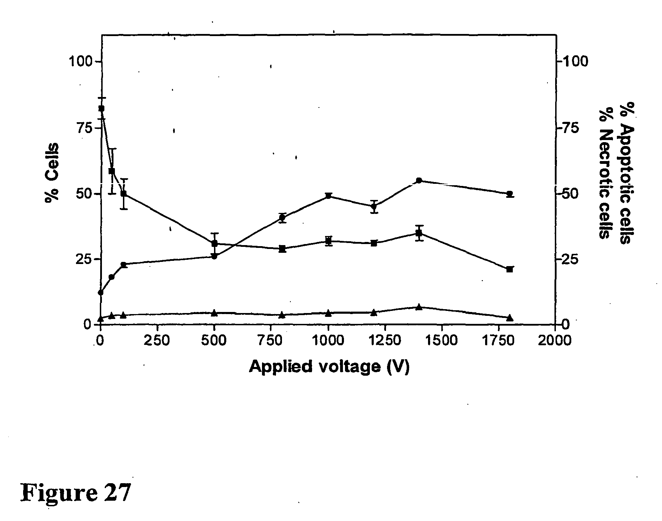

Figure 27 shows the effect of combined treatment of 707 cells with increasing electric field strength (single pulse) and ultrasound at 1.25 W/cm² for 30 seconds using a 3 MHz ultrasound head. Cell concentrations (■) are determined using a haemocytometer and the proportion of apoptotic (●) and necrotic (▲) cells in each population is determined by staining in Annexin V-FLUOS and propidium iodide followed by analysis using flow cytometry. Data reflect mean values ± SEM of three experiments.

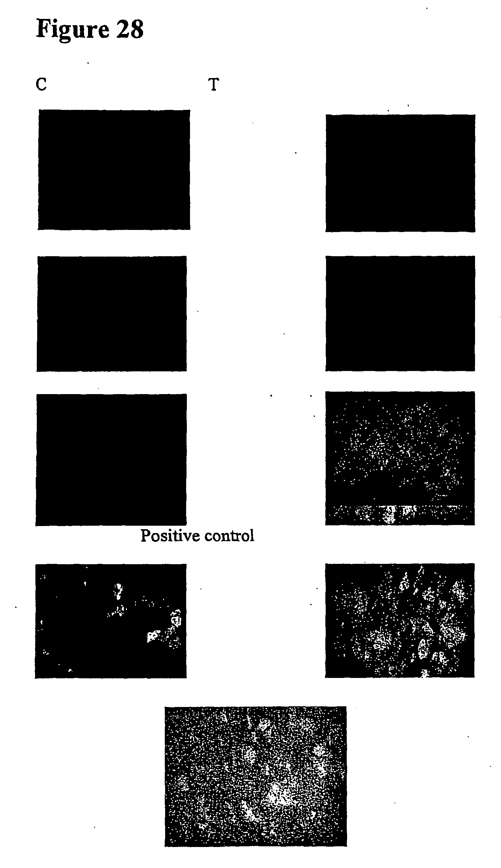

Figure 28. Induction of apoptosis *in vivo,* following treatment with electric fields and ultrasound. Sections from control (Column C) and treated animals (Column T) are stained for apoptosis. Panels from sections harvested at 0, 6, 12, 18 and 24 hours are displayed in descending order in each column. The panel at the bottom of the figure represents a positive control generated by DNAse I treatment of sections prior to staining.

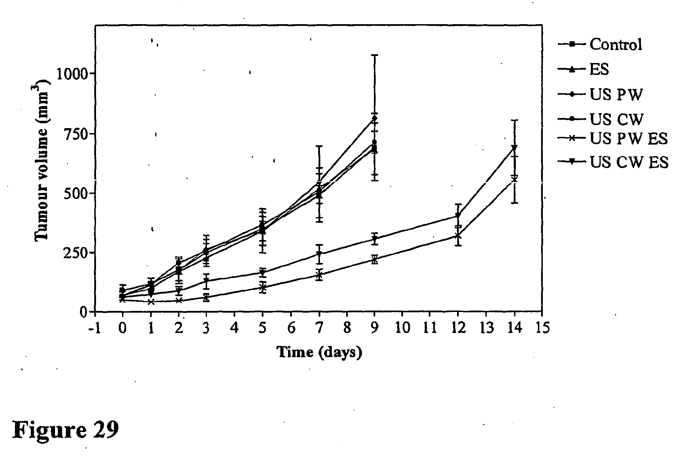

Figure 29 shows the effect of treating tumours with ultrasound prior to electric fields. Groups of animals used in experiments consist of control untreated (■), electrosensitised (▲), ultrasound using pulsed wave (◆), ultrasound using continuous wave (●), pulsed wave ultrasound followed by electric fields (X) and continuous wave ultrasound followed by electric fields (▼). In each group n=4 and the error bars represent + SEM.

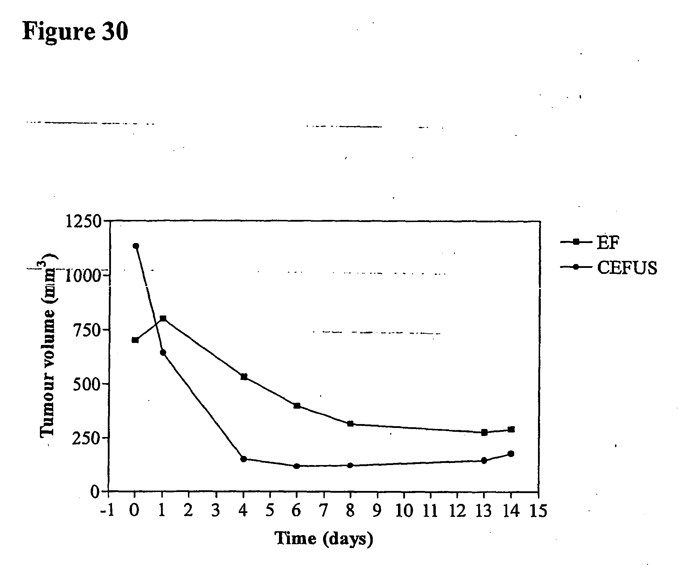

Figure 30 shows the effect of direct electric current (■) and direct electric current together with ultrasound (●) on tumour volume.

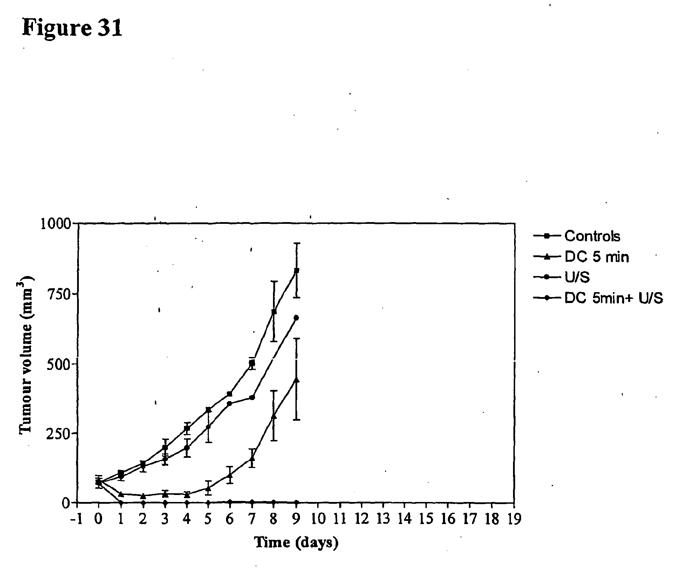

Figure 31 shows the effect of treatment of RIF-1 tumours with ultrasound (●), direct electric current (▲) and combined direct electric current with ultrasound (◆). Control animals are untreated (■). Error bars represent ± SEM where n=2.

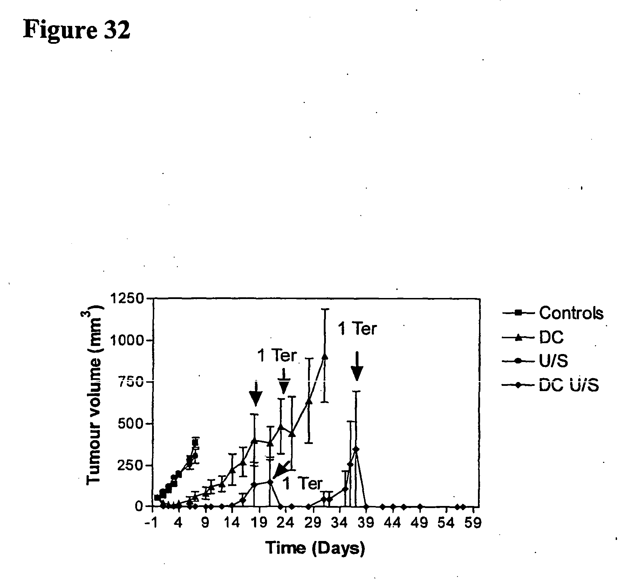

Figure 32 is a graph showing results of treatment of tumours with ultrasound 22 hours after sensitisation with direct current (DC). Each group consists of 4 animals and groups are treated with direct current alone (▲), ultrasound alone (●) and direct current followed, 22 hours later, by ultrasound (◆). Control animals (■) receive no treatment. The bold arrow insets with 'Ter' indicate removal of animals from the experiment. Error bars represent +/- SEM (standard error mean).

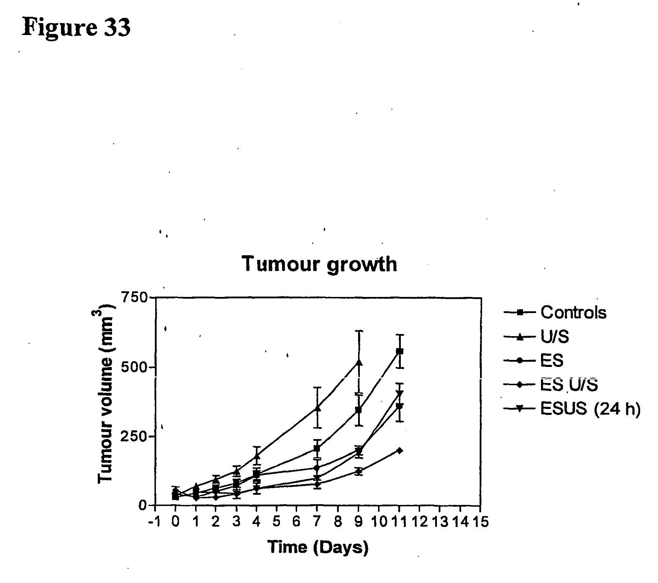

Figure 33 is a graph showing sensitisation of tumours to ultrasound using high-intensity, short-duration square wave electric pulses. Each group consists of 4 animals and each group is treated with electric pulses alone (●), ultrasound alone (▲), electric field treatment followed 24 hours later by ultrasound (V) and electric field treatment followed immediately by ultrasound (◆). Control animals (■) receive no treatment. Error bars represent +/- SEM (standard error mean).

### DETAILED DESCRIPTION

We describe an apparatus capable of ablating a cell through a combination of electric field and ultrasound.

The apparatus is generally capable of achieving disruption or ablation of cells by applying two steps: a sensitisation step followed by a disruption step. In general, sensitisation and disruption may be achieved by exposure of cells to one or more energy sources, including particles, waves or fields. However, in the embodiment of the apparatus described here, the energy sources comprise ultrasound and electric fields, and the apparatus in general comprises an ultrasound delivery component and an electric field delivery component.

The apparatus is capable of generating both an electric field, as well as ultrasound. It therefore comprises at least two modules, an electric field generating module, and an ultrasound generating module. In a preferred embodiment, the apparatus is operable to supply or deliver an electric field to sensitise a cell, and is further operable to supply or deliver ultrasound to a cell which has been sensitised, in order to effect its disruption or ablation.

Thus, in a preferred embodiment, the electric field serves to sensitise a cell, preferably a nucleated cell, to render it more susceptible to disruption by a stimulus. Preferably, the apparatus is capable of sensitising a nucleated cell so that it is rendered more susceptible to disruption by an energy source when compared to a cell, preferably a nucleated cell, which has not been so sensitised. Disruption is achieved by exposure of a sensitised cell to a stimulus at a frequency and/or energy sufficient to disrupt sensitised cells, preferably sensitised nucleated cells, preferably at a frequency and/or energy which is at the same time insufficient to disrupt unsensitised cells. The apparatus comprises in general a component for generating such a disruptive stimulus; in a preferred embodiment, the apparatus comprises a component for generating an ultrasound stimulus.

A cell, preferably a nucleated cell, which has been sensitised by exposure to an electric field from the apparatus (an electrosensitised cell) may therefore be disrupted or ablated by the ultrasound produced by the apparatus. However, it will be appreciated that the apparatus may be used to merely sensitise a cell, which cell is disrupted by a stimulus generated elsewhere. Similarly, the apparatus is capable of disrupting an already sensitised cell, by providing an ultrasound stimulus to the cell.

Each of the delivery components may be driven by signal generating circuitry, as described below.

In a preferred embodiment, the apparatus is operable to achieve selective ablation of cells, in other words, targeted cell disruption or killing.

In a preferred embodiment, the apparatus is operable to generate ultrasound and electric field energy, and exposure of a cell to these (in either order) results in cell death, cell disruption, cell ablation or cell killing. More preferably the apparatus enables cell death etc resulting from apoptosis in the treated cell. Preferably, apoptosis is achieved by administration of electric fields from the apparatus in any combination of (i) high intensity, (ii) short duration, or (iii) as exponential pulses. Most preferably, apoptosis is achieved by administration of electric fields of high intensity, short duration, and as exponential pulses. Alternatively, or in addition, apoptosis may result from application of long duration direct current, as described below.

The term "high intensity" should be taken to refer to electric fields of between about 0.5 kV/cm and 3kV/cm, preferably between about 1 kV/cm and 2kV/cm, more preferably, about 1.3 kV/cm. "Short duration" electric fields in the context of the above passage refer to between about 25 microseconds to 700 ms, preferably between about 25 microseconds to 450 ms, more preferably about 250 ms or 450 ms.

The apparatus as described here is suitable for killing any cell. However, the cell is preferably a nucleated cell, more preferably such a cell from a multicellular organism. Preferably, the cell is comprised in a tissue. In preferred embodiments, the cell is an animal cell, more preferably a mammalian cell, most preferably a primate cell. In highly preferred embodiments, the cell is a human cell.

In preferred embodiments, the cell ablation takes place *in vivo*, i.e., in the body of the organism. However, the apparatus may equally be used *in vitro* or *ex vivo* for cell ablation.

Preferably, the apparatus is used to ablate a tumour cell, a cancer cell or a diseased cell, or an otherwise abnormal or unwanted cell. Preferably, the cell to be ablated, etc is in a tissue or tissue mass, for example, a growth such as a cyst or a tumour tissue or tumour such as a solid tumour. The apparatus is therefore capable of treating and ablating cells as well as tissues comprising cells. The tumour may be a benign or a malignant tumour. Thus, the apparatus described here may be used to treat benign tumours for which traditional tumour therapies (e.g., chemotherapy, radiotherapy, etc) are contraindicated. The apparatus may also be used as an adjunct or substitute for any kind of conventional surgery to remove unwanted tissues.

In other embodiments, the apparatus is used to ablate or disrupt or kill a cell, for cosmetic purposes, i.e., to improve appearance. In some embodiments, these are purely cosmetic purposes, i.e., non-medical purposes, for the improvement of image or appearance of an individual with no medical benefits. For example, the apparatus may be used to remove a mole, a birthmark a vascular birthmark, a salmon patch (nevus simplex), a strawberry hemangioma, a port wine stain (nervus flammeus), a blemish, a freckle, a wrinkle, a scar tissue, etc from the skin for the purposes of improving the appearance of an individual.

Salmon patch (nevus simplex) is a flat patch of pink or red skin, often small, usually with poorly defined borders. Salmon patches are seen in 30 percent to 40 percent of newborns. They typically are found at the nape of the neck ("stork bite"), on the forehead between the eyebrows ("angel's kiss") or on the eyelids. Often, they are more noticeable with crying or changes in temperature. Strawberry hemangioma is a raised bright red spot, often small, usually soft and compressible, with well-defined borders. It occurs most commonly on the face, scalp, chest or back. It may be present at birth but more often appears during the first one or two months of life. Strawberry hemangiomas occur in 1 percent to 3 percent of infants. In rare cases, they interfere with vital organs or are associated with life-threatening complications. Port-wine stain (nevus flammeus) is a flat patch of purple or dark red skin, often large, usually with well-defined borders. It typically occurs on one side of the face or neck and is present at birth. Other conditions such as acne may also be treated by the apparatus described here, for improving appearance.

The apparatus as described here is capable of being used to ablate cells or tissues in any multicellular organism, and is advantageously applied to organisms having distinct tissues which may be targeted for electrosensitisation. Advantageously, the organism is a mammal. Preferably, the target tissue is a tumour tissue, more preferably a solid tumour tissue. More preferably, the target cell, tissue or tumour etc is treated *in situ* in the organism. However, the target cell, tissue or tumour etc may be removed from the organism, for example, by surgery, and treated *ex vivo*. In some embodiments, the removed explant, after being treated to remove unwanted cells, etc, may be re-introduced into the body of the organism (or indeed any other organism).

Preferably, where the tissue is a tumour tissue, treatment of the tissue with ultrasound and electric field from the apparatus described here leads to partial or complete remission. Thus, in a particular embodiment, treatment with the apparatus leads to no significant cell growth of tumour cells (at the treated site or preferably in the body of the organism) within a relevant period. Such a period is preferably 1 day, 1 week, 1 month, 2, 3 4, 5 or 6 months, or even longer, for example, a year, two years, five years, 10 years, 20 years, etc.

Preferably, the sensitisation procedure is carried out in the absence of foreign material, for example, material intended for incorporation into the cell. Thus, for example, where electrosensitisation is followed by application of ultrasound, the electrosensitisation procedure is carried out in the absence of foreign material, for example, material intended for incorporation into the cell.

However, and as described in further detail below, other agents (such as cytotoxics and cytokines), may be applied to the cells after administration of the sensitiser (e.g., electric field) and/or after administration of the disrupter (e.g., ultrasound) to promote cell death. Such cell-death facilitating agents may be administered to prior to the disrupter (e.g., ultrasound), i.e., to sensitised cells. We describe a specific embodiment in which the electric field is delivered to the cell or tissue, or in the vicinity of these, using one or more hollow needles. Such hollow needles may serve as a conduit to deliver a cell-death facilitating agent to the cell or tissue, from a reservoir housed in the apparatus.

Furthermore, the cell-death facilitating agents may be applied together with, or subsequent to, administration of the disrupter, for example, the ultrasound, by the use of suitable hollow ultrasound delivery components.

The cell-death facilitating agents may be applied alone or in combination with each other; furthermore, the cell-death facilitating agents may be applied in the form of cells expressing the agents.

The apparatus described here is preferably capable of treating or disrupting a cell, preferably a nucleated cell. The cell may therefore comprise a nerve cell, a muscle cell, an epidermal cell, a capillary cell, an epithelial cell, an endothelial cell, etc. The cell may be normal, diseased, infected, cancerous, or otherwise abnormal. Any of these cells and others may be disrupted by use of the apparatus described here.

More preferably, the cell is part of a tissue mass in the organism and a proportion of the tissue is sensitised (e.g., electrosensitised or ultrasound sensitised, preferably ultrasound sensitised). The proportion of the tissue which is sensitised will vary, but advantageously, substantially all of the tissue becomes sensitised by electricity or ultrasound. For example, about 50%, 60%, 70%, 80%, 90% or 100% of the cells of the tissue are sensitised by use of the apparatus as described here.

Electric field energy is preferably administered substantially as described below, by use of an electric field delivery component comprised in the apparatus. The electric field delivery component is connected to receive a signal generated by electric field generating circuitry. The electric field which is generated by the apparatus preferably comprises one or more electric pulses of from about 1 Volt/cm to about 10 kVolts/cm under *in vivo* conditions. Instead of or in addition to the pulses, the apparatus may be adapted to deliver an electric field in a continuous manner. The electric pulse may be applied for between 1 µs and 700 milliseconds, preferably between 1 µs and 500 milliseconds, more preferably between 1 µs and 100 milliseconds. The electric field may be applied continuously or in a pulsed manner for about 5 minutes or more.

The apparatus further comprises an ultrasound delivery component, which is connected to receive a signal generated by ultrasound signal generating circuitry. The apparatus is preferably capable of generating and delivering ultrasound at a power level of from about 0.05W/cm² to about 100W/cm². Diagnostic or therapeutic ultrasound may be used, or combinations thereof. A particular embodiment of the apparatus comprises a separate ultrasound generating component capable of generating ultrasound at diagnostic levels, in conjunction with a further ultrasound generating component capable of generating ultrasound at therapeutic levels to effect disruption. The diagnostic ultrasound may be used for analysis of the tissue before, during or after disruption, as described in further detail below.

The ultrasound signal generating circuitry and the electric field signal generating circuitry may be capable of generating a number of different waveforms, such as continuous wave and pulsed wave. The ultrasound signal generating circuitry and the electric field signal generating circuitry are controlled by a controller, described in more detail below.

The controller may control the delivery of ultrasound and electric field, so that they are delivered simultaneously or separately. Thus, the apparatus is capable of performing the sensitisation and disruption steps separately or simultaneously. Electrosensitisation and ultrasound disruption, or ultrasound sensitisation and disruption by electric field, may be simultaneous or separate. Where the steps are performed separately, the apparatus may be capable of performing them in any order. For example, the ultrasound may be administered before the electric field. Advantageously, however, the electrosensitisation step precedes the ultrasound disruption step.

Furthermore, the apparatus is capable of delivering single or multiple applications of electric field, as well as single or multiple applications of ultrasound, in any order and in any combination. Thus, the apparatus may be configured so that it enables multiple cycles of sensitisation, followed by disruption (i.e., S + D, S + D, S + D..., for example, ES + US, ES + US, ES + US... where ES is electrosensitisation and US is ultrasound). Two or more applications of sensitisation may be followed by a single disruption (i.e., S + S... + D). Furthermore, two or more applications of electric field may be followed by a single application of ultrasound (i.e., ES + ES... + US), or *vice versa* (i.e., US + ES + ES...). A single electrosensitising field may be applied, followed by two or more ultrasound applications (e.g., ES + US + US...) and *vice versa* (i.e., US + US + ES...). Multiple electrosensitising fields may be applied followed by multiple ultrasound applications (ES + ES... + US + US...), or multiple ultrasound applications by multiple electrosensitising fields (US + US... + ES + ES...). In each of the above, the ultrasound and/or the electric field may be delivered as single or multiple continuous applications, or as pulses (pulsatile delivery). The above protocols may be combined with each other.

We also provide for a cell, preferably a nucleated cell, which has been sensitised by being exposed to an electric field or ultrasound delivered by the apparatus described here. Thus, we provide a cell, preferably a nucleated cell, which has been rendered sensitive to a stimulus by exposure to ultrasound or an electric field. Included are cells (preferably nucleated cells) treated with ultrasound, as well as cells (preferably nucleated cells) treated with an electric field, the treatment rendering the cells sensitive to a stimulus. Thus, we provide in particular, nucleated cells which are rendered sensitive to ultrasound by exposure to an electric field from the apparatus, as well as nucleated cells rendered sensitive to an electric field by exposure to ultrasound from the apparatus.

The apparatus, methods, products etc described here will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

### APPARATUS FOR ABLATING A CELL

The structure of a device for destroying, ablating or disrupting a cell is described under "Apparatus" with reference to the figures. Particular aspects of the apparatus are then further described in detail under the headings "Electric Field Signal Generating Circuitry (EFG)", "Electric Field Delivery Component (EFD)", "Therapeutic Ultrasound Signal Generating Circuitry (USG)", "Therapeutic Ultrasound Signal Delivery Component (USD)", "Diagnostic Ultrasound Signal Generating Circuitry (DG) and Diagnostic Ultrasound Delivery and Scanning Component (DDS)" and "Controller".

Finally, background information relating to cell ablation, including the processes of sensitisation and disruption are set out under the headings "Sensitisation", "Electrosensitisation", "Ultrasound", "Low Intensity Sensitisation and Disruption", "Agents which Facilitate Cell Death", "Cytotoxics", "Cytokines", "Apoptosis" and "Assays". These are supported by detailed Examples showing that application of an electric field and ultrasound to a cell such as a nucleated cell, in either order, causes the cell to be disrupted. The inclusion of such background material enables the process to be understood and hence the apparatus to be optimised for its purpose, i.e., delivery of ultrasound and electric field to a cell in order to disrupt or ablate it.

### APPARATUS

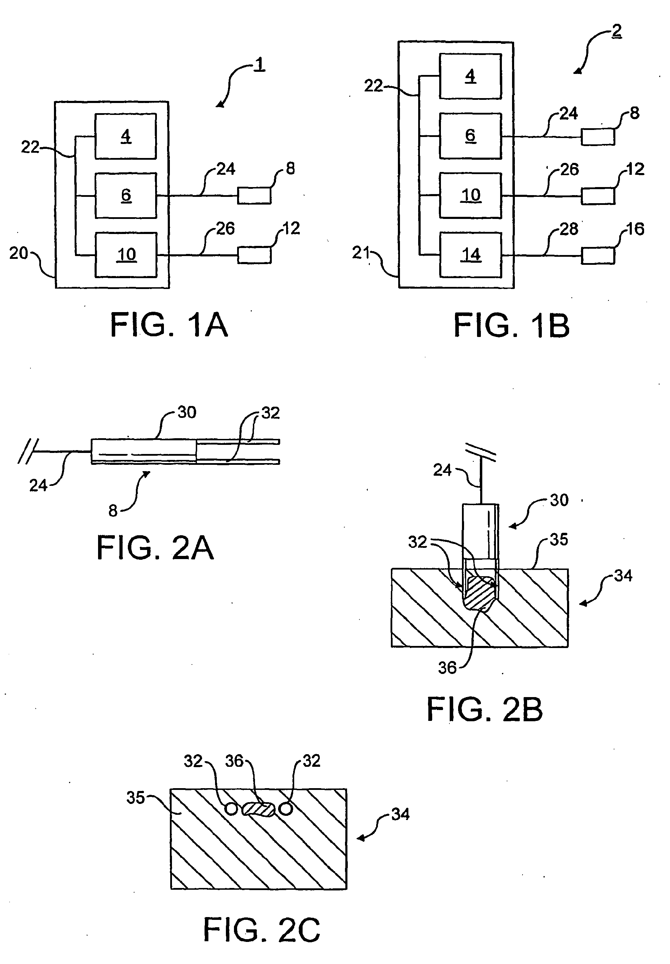

Figure 1A is a highly schematic diagram showing a cell ablation or tumour therapy device 1 according to an embodiment of one aspect of the invention. The tumour therapy device 1 comprises a controller 4, electric field signal generating circuitry (EFG) 6, an electric field delivery component (EFD) 8, therapeutic ultrasound signal generating circuitry (USG) 10 and a therapeutic ultrasound delivery component (USD) 12. Whilst the controller 4 may be an application specific unit, in this example it comprises a general purpose computer configured to perform the required tasks. The controller 4, the EFG 6 and the USG 10 are interconnected by signal and control cabling 22 and collectively form a main unit 20. Some or all of the components comprising the main unit may be contained within a single housing, for example to minimize external cabling or increase portability. In other embodiments, aspects of the controller's functionality may be devolved to control sub-units within the EFG or USG. The EFD is coupled to the EFG by control and signal cabling 24 and the USD is coupled to the USG by control and signal cabling 26.

Figure 1B is a highly schematic diagram showing a tumour therapy device 2 according to another embodiment of one aspect of the invention. As with the tumour therapy device 1 shown in Figure 1A, and using the same reference numerals to denote functionally similar features, the tumour therapy device 2 shown in Figure 1B also comprises a controller 4, EFG 6, an EFD 8, USG 10 and USD 12. These components and their interconnections are similar to and will be understood from the description above.

However, the tumour therapy device 2 shown in Figure 1B further comprises optional diagnostic ultrasound signal generating circuitry (DG) 14 and a diagnostic ultrasound delivery and scanning component (DDS) 16. The DDS is connected the DG by control and signal cabling 28. The controller 4, the EFG 6, the USG 10 and the DG 14 are interconnected by signal and control cabling 22 and collectively form a main unit 21. As before, some or all of the components comprising the main unit may be contained within a single housing, for example to minimize external cabling or increase portability. In other embodiments, aspect of the controller's functionality may be devolved to control sub-units within the EFG or USG.

Each of the main components of the tumour therapy device 2 shown in Figures 1A and 1B, including the optional diagnostic ultrasound signal generating circuitry (DG) and diagnostic ultrasound delivery and scanning component (DDS), are now described in turn.

### ELECTRIC FIELD SIGNAL GENERATING CIRCUITRY (EFG)

The EFG 6 is operable to generate an electric field signal which is supplied to the EFD 8 via cabling 24 to provide an electric field at a treatment site in the vicinity of the EFD. The EFG may be operable to provide a wide range of one or more electric field signals, for example, oscillatory, steady state, pulsed, pulsed exponential decay, generalized periodic or randomized waveforms, or a combination thereof. The supplied waveforms can display a range of characteristic timescales and amplitudes selectable dependant on the requirements of a particular application.

The electric field signal generation circuitry may function in any known manner to generate the desired electric field signal. For example, the EFG may comprise one or more programmable arbitrary waveform generators, such as the Wavetek 295 (Wavetek Corp., San Diego, California, USA), coupled to a suitable voltage and/or current amplifier, and under control of the controller 4. Alternatively, if a smaller number of specific waveforms are required, the EFG may comprise a device such as the BTX ECM 630 or BTX ECM 830 (BTX Division of Genetronics, Inc., San Diego, California, USA), providing exponential decay and square wave electric field signal pulses respectively, operating under control of the controller 4. In the example shown in Figure 1B, the EFG 6 is operable to supply an output electric field signal with a characteristic timescale selectable between 1 msec and 20 minutes, and at an amplitude corresponding to a voltage selectable between 1 and 3000 V, for example.

Depending on the specific geometry of the EFD, this voltage range may preferably generate an electric field of between 1 V/cm and 10 kV/cm at the treatment site. Thus, the electric field may have a strength of 1 V/cm, 2 V/cm, 3 V/cm, 4 V/cm, 5 V/cm, 6 V/cm, 7 V/cm, 8 V/cm, 9 V/cm, 10 V/cm, 20 V/cm, 50 V/cm, 100 V/cm, 200 V/cm, 300 V/cm, 400 V/cm, 500 V/cm, 600 V/cm, 700 V/cm, 800 V/cm, 900 V/cm, 1 kV/cm, 2 kV/cm, 5 kV/cm, 10 kV/cm, 20 kV/cm, 50 kV/cm or more. More preferably from about 0.5 kV/cm to about 4.0 kV/cm. However, the electric field strengths may be lowered where the number of pulses delivered to the target site are increased. Thus, pulsatile delivery of electric fields at lower field strengths is envisaged. Depending on the therapy requirements of the application, the EFG may supply more than one dependent or independent electric field signal, for example the EFG may supply the EFD with multiple signals of differing phase, or with multiple completely independent signals with differing waveforms and/or different times of application.

The EFG may be capable of supplying the electric field in either a unipolar mode and also be equipped with a polarity alternating capability.

The controller 4 may be configured so that it is capable of determining the amount of energy deposited by the electric field at the treatment site. In this example, this is achieved by monitoring the current flow to the EFD in combination with the selected signal amplitude. Alternatively, the impedance of current flow paths within the treatment site can be measured and employed in combination with either the monitored current flow or the selectable signal amplitude. The EFD may also include a temperature sensor (not shown) which measures the temperature in the vicinity of the treatment site.

These parameters can be used to from an electric field discharge profile (i.e. details of voltage, current, impedance, temperature etc.) during operation of the EFG. Electric field discharge profiles may be stored by the controller 4 for subsequent analysis. Furthermore, based on the electric field discharge profile parameters (in addition to similar operational parameters relating to the USG and diagnostic information derived from the DDS) the EFG functioning can be responsively controlled by the controller 4. In other embodiments, aspects of the feedback control may be internal to the EFG. Whilst not shown here, the EFG may also be capable of monitoring the moisture/lipid/salinity ratio content in the vicinity of the EFD using techniques known in the art. This allows impedance measurements to be employed to help predict localised electric field density at the treatment site.

### ELECTRIC FIELD DELIVERY COMPONENT (EFD)

In operation, the EFD 8 serves to interface the electric fields generated by the EFG 6 with tissue at the treatment site and, in the example described here, the EFD is interchangeably coupled to the EFG such that different EFD geometries and configurations can be employed as required for specific therapy applications. Thus the apparatus may comprise one or more modular components, which are detachable and interchangeable, for further flexibility.

The EFD operates to deliver the electric field to the treatment site, and will generally comprise one or more contact electrodes (also known as "electrical contacts") for coupling the signal supplied by the EFG to the treatment site. The terms "contact electrode" and "electrical contact" should be understood to be synonymous with each other.

For example, the EFD may comprise needle electrodes for insertion into the tissue at the treatment site. The needles may be formed from any suitable material, e.g., a hypoallergenic material. Such materials may include any combination of stainless steel, platinum or platinum coated structures, diamond-like carbon-coated electrodes or other such suitable materials as is known in the art. Alternatively, the EFD contact electrodes may comprise conductive parts of a contact pad attached to the tissue surface in the vicinity of the treatment site. Other EFD configurations may comprise a combination of conductive pads and needles.

It will be appreciated that the term "needle, in the context of this document, should be taken to include any rod-shaped member, such as a pin, a rod, or a pen. Accordingly, the electrical contacts may take such a general form, and need not necessarily have a pointed or sharp end.

It is also noted that not all of the contact electrodes need be in the immediate vicinity of the contact site. For example, if required by a particular therapeutic application, the EFD might comprise a single contact electrode at the treatment site and a more distant second contact electrode, which might, for instance, be held at ground potential.

Figure 2A is a schematic side view detailing an EFD configuration suitable for use in the tumour therapy device 1 shown in Figure 1A or a tumour therapy device 2 shown in Figure 1B. The EFD 8 shown in Figure 2A comprises an EFD body 30 and a pair of parallel needles 32. The EFD body serves to support the needles and also contains sensors to determine the parameters discussed above using standard techniques. Signals from the EFG are routed from the cabling 24 to the needles 32 via connections (not shown) internal to the EFD body 30. In operation, the needles 32 behave as a pair of electrical contacts which are inserted into the tissue surrounding the treatment site and an electric field generated by the EFG is applied as required. The separation of the needles can be chosen based on the size of the treatment site and/or the required electric field strength for a given voltage supplied by the EFG.

Figures 2B and 2C are schematic views showing two example orientations of the EFD shown in Figure 2A when in use. In Figure 2B, the needles 32 are introduced into the tissue 34 in a direction which is generally perpendicular to the tissue surface 35 such that the needles are positioned either side of a treatment site 36, e.g. a tumour. In Figure 2C, the needles 32 are introduced into the tissue 34 in a direction which is generally parallel to the tissue surface 35 such that the needles are positioned either side of a treatment site 36. The preferred orientation will depend on access to, and the geometry of, the treatment site and other angles of introduction will be appropriate in other situations.

For some applications, for instance if the treatment site 36 is particularly close to the surface of the tissue 34, or the tissue to be treated upstands from the surface (e.g., where a mole is being treated), the needles 32 comprising the electrical contacts need not be inserted into the tissue but merely held against it. However, for such applications an electrical contact pad electrode, as described further below, may be preferred.

Furthermore, it will be evident that straight electrodes need not be used, and that bent or curved or kinked electrode configurations may be more suitable for some purposes, for example, those described above.

The EFD electrical contact configuration shown in Figure 2A (i.e. a single pair of parallel needles) is relatively simple. Other EFD electrical contact configurations, such as those described further below, will be appropriate in other situations. For instance, if a treatment site is distributed, an array of suitably driven electrical contacts can provide a more uniform electric field across the treatment site than would be achieved with a single pair of electrodes. In other cases, a localized electric field might be required against a lower background electric field, and electrical contact configurations in the EFD can be appropriately designed based on the well understood laws of both electrodynamics and electrostatics.

Aspects of the EFD which are not specifically described in the examples below, such as the cables coupling the EFD to the EFG, will be similar to and understood from the description given above. Some of the following configurations will be widely applicable, whilst others will be more specifically so. Certain therapy applications may require other EFD electrical contact geometries to be designed and these may or may not be based on combinations of features shown in following examples. As noted above, the geometric scale of the EFD may also by based on the specific applicational requirements. Various other electrode arrays are known in the art (see for example US 5,720,921, WO 99/62592, WO 98/56893, US 6,041,252 and US 5,873,849) and the device described here may employ any one, or more, of such electrode configurations.

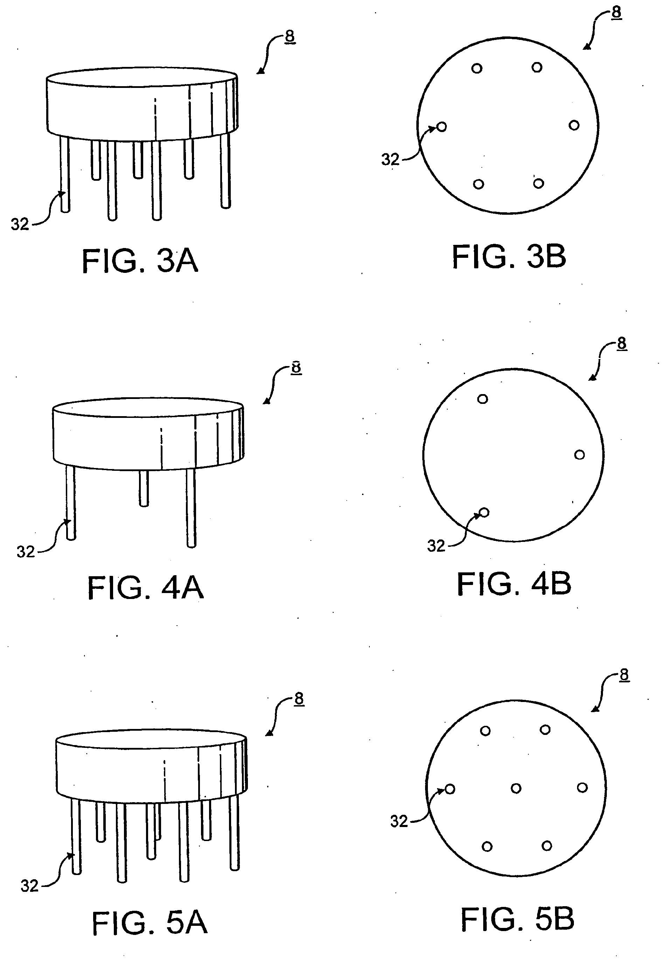

Figure 3A is a schematic perspective view of an EFD 8 with an electrode configuration comprising of an array of six parallel needles 32. Figure 3B is a schematic plan view of the EFD shown in Figure 3A and shows the needle arrangement in a plane perpendicular to their axes of extent. The needles 32 are respectively coupled to the EFG such that opposing needle pairs provide electrical contacts for the application of three independent electric field generation signals from the EFG. As in all of the examples given below, the sequence in which signals are applied to the needle electrodes, e.g. simultaneously, in-turn, or phase delayed etc., may be controlled either by the controller 4 or the EFG 6. Different numbers of pairs of needles can be used and similarly arranged in a polygon array, e.g. 2, 4 or 5 pairs of needles in a cross, octagon or decagon pattern, depending on the details of the area of tissue to be treated. In other examples, the needles will not be regularly arranged.

Figure 4A is a schematic perspective view of an EFD 8 with an electrode configuration comprising of an array of three parallel needles 32. Figure 4B is a schematic plan view of the EFD shown in Figure 4A and shows the needle arrangement in a plane perpendicular to their axes of extent. In this example, the needles 32 are coupled to the EFG such that electric fields can be generated between any two of the three needles (or indeed, a voltage can be applied simultaneously to all three needles) to provide a desired electric field configuration at the treatment site.

As in the example EFD shown in Figures 4a and 4b, in which the electric field can be generated between any combinations of the contact electrodes, it will be understood that electric fields can also be generated between any combinations of the contact electrodes in an EFD containing more than three contact electrodes. For instance, whilst by way of example only, the six needles of the EFD shown in Figures 3a and 3b are paired to form three independent circuits, a similar arrangement of needles could also be employed with an EFG configured to apply a signal to any combinations of the needles to provide a desired electric field at the treatment site.

Figure 5A is a schematic perspective view of an EFD 8 with an electrode configuration comprising of an array of seven parallel needles 32 wherein an outer ring of six needles surrounds a central needle. Figure 5B is a schematic plan view of the EFD shown in Figure 5A and shows the needle arrangement in a plane perpendicular to their axes of extent. As described above, signals can be applied to any number of the needles in any combination. For instance, an electric field can be applied between the central needle and a single one of the external needles, between the central needle and two or more of the outer needles, or between combinations of the outer needles only. The direction of the applied electric field can also be varied.

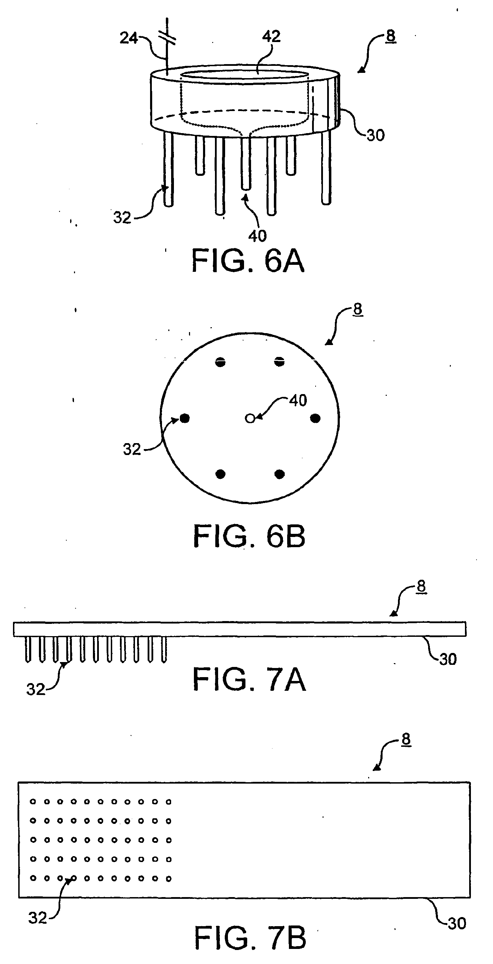

Figure 6A is a schematic perspective view of an EFD 8 with an electrode configuration comprising of an array of six parallel needles 32 surrounding a hollow-core central needle 40. Figure 6B is a schematic plan view of the EFD shown in Figure 6A and shows the needle arrangement in a plane perpendicular to their axes of extent. The hollow-core central needle 40 is in fluid communication with a reservoir 42 within the EFD body 30. The reservoir 42 is coupled to a syringing device (not shown) such that fluid can be forced from, or drawn into, the reservoir 42 through the hollow-core central needle 40. This allows, for example, the administration of a cell death facilitating agent, or saline to flush out dead cells or other drug delivery to the treatment site, or sample extraction from the treatment site during therapy. In practice, the reservoir 42 may simply comprise a sealed flow chamber to allow fluid communication between the hollow core needle 40 and a conventional syringe or other vessel.

Where an EFD provides for administration of a cell death facilitating agent, saline or other drug delivery to the treatment site, or sample extraction from the treatment site, this may be performed under the control of the controller. For instance, for applications involving the administration of a cell death facilitating agent, the tumour therapy device may optionally further include a reservoir containing the cell death facilitating agent which is administered (via tubing to the EFD) by a mechanical pump (or by otherwise pressurising the source of cell death facilitating agent) under the control of the controller 4. For example, if appropriate for a particular therapy application, the controller might be configured to administer the cell death facilitating agent to the treatment site at the same time that an electric field is applied. In other situations it may be appropriate to avoid administering the cell death facilitating agent to the treatment site at the same time that an electric field is applied. Under the control of the controller, the administration of the cell death facilitating agent can be governed in any manner which is suited to the requirements of a particular therapy and which may be responsive to details of the applied electric field and/or ultrasound. Similarly, the controller could be configured to instigate pumping of saline to flush away dead cells, or to draw samples from the treatment site, at any appropriate stage of the therapy.

Electric fields can be applied as desired between any of the needles, including the central hollow core needle. In other configurations of EFD there may be more than one hollow needle. For example, if some or all of the outer ring of six needles shown in Figures 6A and 6B were to be hollow and in fluid communication with the reservoir 42, fluid could be injected into (or drawn from) a number of locations within or surrounding the treatment site. Alternatively, the EFD body 30 might contain multiple separate reservoirs such that different fluids could be injected into, or different samples taken from, different locations within or surrounding the treatment site.

Figure 7A is a schematic section view of an EFD 8 with an electrode configuration comprising of an matrix of, in this example, fifty-five parallel needles 32 arranged in a regular rectangular array. Figure 7B is a schematic plan view of the EFD shown in Figure 7A and shows the needle arrangement in a plane perpendicular to their axes of extent. As in other examples, electric fields can be applied between any combinations of the needles 32 to provide a desired electric field at the treatment site, and be controlled by the controller 4 or EFG 6.

In the examples given above, the needles comprising the contact electrodes are uninsulated along their axes of extent. In some situations, for instance where electrical contact is required with a treatment site near to tissue with which electrical contact should be avoided, some or all of the needles may be partially insulated.

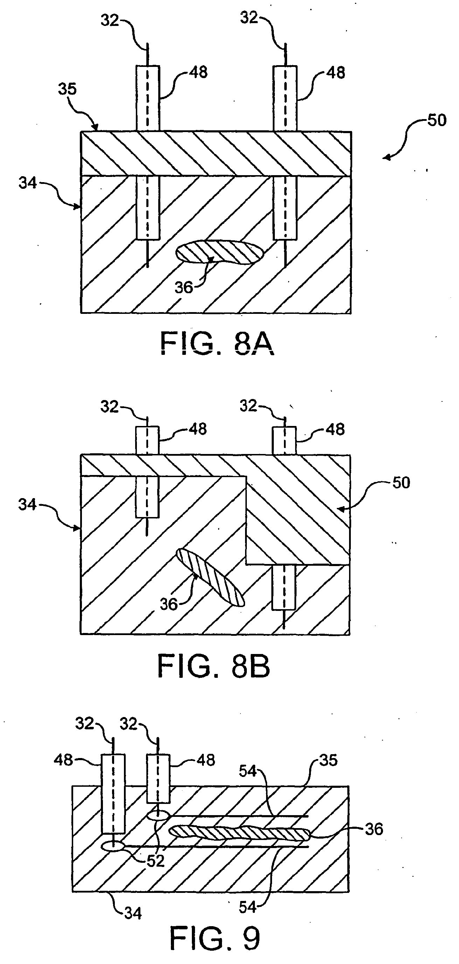

Figure 8A shows a schematic side view of two needle electrodes 32 inserted into tissue 34 to access a treatment site 36. In this example, a layer of essential tissue 50 (i.e. one which must not be held in electrical contact with the needles) is located just below the tissue surface 35. The needles 32 are electrically insulated along a portion of their length by insulators 48. The insulators may be made from any appropriate non- or poor-conductor, for example PTFE, which can be affixed to the needles 32.

In a preferred embodiment, the degree of insulation of the needle is variable and may be adjusted. This may be achieved by applying different thicknesses of insulating material, or by adjusting the extent of the needle which is covered by the insulating material. Thus, the needle may be covered by insulating material along a variable portion of its length. Alternatively, or in addition, the insulating material may form a sleeve which covers the needle, the length of the sleeve being adjustable or extendible. Any suitable insulating material, for example, material which is deformable, may be used for this purpose.

A conduction path is maintained within the insulator 48 such that the electric field signals from the EFG are coupled to the lower portion of the needles 32 within the tissue 34 and apply an electric field to the treatment site 36. The insulators 48 ensure no current flows within the essential tissue layer 50 and so avoids possible damage to it. The feature of insulating some or all of the needles along a portion of their length can readily be used in combination with any needle configuration, such as those discussed above.

Figure 8B is a schematic side view of another example in which a region of essential tissue 50 is protected from current flow driven by the electric field. Because of the particular geometry of the treatment site and essential tissue in this example, the needles 32 are of different lengths. As above, the needles are insulated with insulators 48 to ensure electric field signals from the EFG drive current flow at the treatment site 36 but not within the essential tissue 50.

In addition to directly providing the source for the electric field within the vicinity of the treatment site, needles can also be used to couple the signal from the EFG to previously implanted contact electrodes.

Figure 9 is a schematic section view of two needles 32, again electrically insulated along portions of their lengths by insulators 48, which have been inserted into tissue 34 to make contact with receiver contacts 52 connected to contact electrodes 54 as shown in the figure. This arrangement is appropriate, for example, where a treatment site 36 has a substantial extent in a plane parallel to the tissue surface 35 but where access such as shown in Figure 2C is not viable.

The contact electrodes 54 in this example are surgically implanted into the tissue 34 where they may remain for an extended period to allow regular and repeated treatment. The surgical implants may be biodegradable to avoid the need for their surgical removal. During a session of treatment, needles 32 are inserted into the tissue 34 to make contact with receiver contacts 52. The receiver contact size will be determined by the ability to reposition the needles in subsequent insertions. The needles may be spring loaded to facilitate electrical contact. Once contact is made, the electrodes 54 are coupled via the needles 32 to the EFG and electric fields can be applied to the treatment site as required.

EFD configuration such as those described above need not operate with the EFD outside of the tissue, but could be bodily introduced into the tissue by mounting suitably small examples on a laparoscopic, endoscopic, bronchoscopic or any key-hole surgery device to facilitate access to an internal treatment site.

As noted above, the contact electrodes of the EDS 8 may also comprise conductive parts of a contact pad. Such configurations are likely to be most appropriate where the treatment site is at or near to the tissue surface.

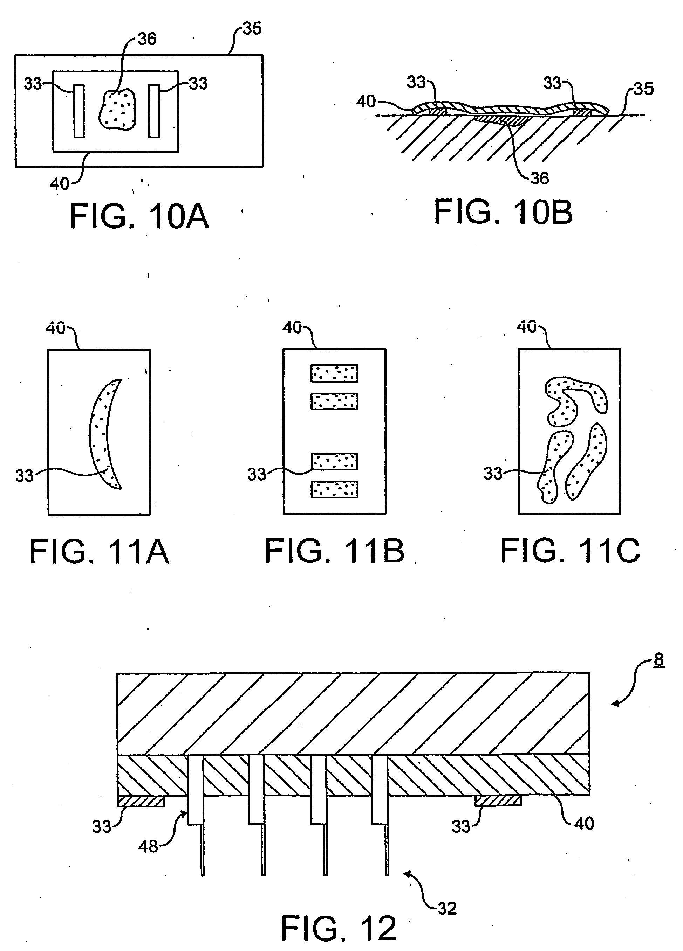

Figure 10A is a schematic plan view of a contact pad 40 of an EFD (main EFD body not shown) applied to a tissue surface 35. Figure 10B is a schematic section view further detailing the contact pad 40 and taken in a plane perpendicular to the plan view shown in Figure 10A. The contact electrodes comprise two elongate conductive parts 33 fixedly attached to the contact pad 40 and which can be held in contact with the tissue surface on either side of the treatment site 36. The contact pad may be self-adhesive to help maintain contact with the tissue surface 35. An electric field signal generated by the EFG is then applied to the treatment site as required via cabling (not shown) within the EFD body and contact pad. As above, the separation of the contact electrodes will be chosen based on the size of the treatment site and/or the required electric field strength as a function of the voltage supplied by the EFG.

In addition to applying an electric field directly to a tissue surface, an additional benefit of a contact pad based EFD is that the conductive parts can be formed using many conventional printed circuit techniques, such as, for example, standard lithography and etching, stencilling, screen printing and other standard printing methods used in conjunction with a conductive ink (including ink-jet or laser-jet printing with a conductive toner), or even direct sketching with a conductive-ink pen. This allows for highly case specific contact electrode geometry configurations to be easily realized. For example, in using a printed circuit contact pad, there is no difficulty in providing an electrode configuration containing multiple irregularly shaped contacts appropriate to suit a highly specific treatment site geometry while minimizing undesired exposure of surrounding tissue to electric fields.

The contact pad, whether or not printed with circuitry, may preferably comprise material which has low ultrasound absorption, in order that the ultrasound not be attenuated significantly.

Figures 11A, 11B and 11C schematically show in plan view three example electrode configurations which are easily achievable with a printed circuit contact pad. In each case the electrode configuration can be coupled to the EFG as described above and different combinations of electric field applied. It will be appreciated that there is essentially no limit to the number of contact electrode configuration which can be formed.

Figure 12 is a schematic section view of an EFD 8 which includes several of the features described above. The EFD 8 comprises a main body to support and couple electric field signals from the EFG to the contact electrodes. The contact electrodes comprise two conductive parts 33 mounted on an adhesive pad 40 and four needles 32, which are insulated by insulators 48. As described above, electrical fields can be applied between any chosen combination of contact electrodes 32, 33 to provide a desired pattern of electric field at the treatment site.

Whilst we have described the above EFD electrode configurations in the context of the tumour therapy device 2 shown in Figure 1B, it will be appreciated that the electrode configurations described may also be used in other applications. For example, they may suitably be employed for electroporation, electrofusion, acupuncture, electrotherapy, etc. Accordingly, we disclose an electric field delivery component comprising a number of contact electrodes, at least one of which is hollow, for insertion into a tissue mass, wherein said number is, for example, 1, 2, 3, 4, 5, 6, 7, 8, 9, or greater than 10, 20, 30, 40 or 50.

We further disclose such an electric field delivery component wherein the contact electrodes are positioned at the vertices of a regular polygon, for example, a triangle, a square, a pentagon, a hexagon, a heptagon etc. We also disclose an electric field delivery component wherein the number of contact electrodes are positioned at the vertices of a regular polygon and in which one or more further contact electrodes are located within the circumference of the polygon, one or more of the further contact electrodes may also be hollow.

In addition, we disclose an electric field delivery component comprising a number of contact electrodes, at least one of which is hollow, and in which the contact electrodes are arranged to form a grid of contact electrodes. Any of the preceding contact electrodes may be insulated along at least a portion of their length. We also disclose an electric field delivery component comprising a number of contact electrodes formed from conductive parts of a contact pad. In addition, we further disclose an electric field delivery component comprising combinations of the above described contact electrodes for insertion into a tissue mass and contact electrodes formed from conductive parts of a contact pad. We also disclose an electric field delivery component comprising a contact pad with low absorption of ultrasound.

### THERAPEUTIC ULTRASOUND SIGNAL GENERATING CIRCUITRY (USG)

The USG 10 is operable to generate an ultrasound signal which is supplied to the ultrasound delivery component (USD) 12 via cabling 26 to provide ultrasound in the vicinity of the USD. In operation, the vicinity of the USD will generally be a treatment site at least partially overlapping with that of the ESD discussed above. The geometry of the USD will determine the distribution of ultrasound energy at the treatment site as discussed further below.

The USG may be operable to provide a wide range of ultrasound signals, for example, the frequency of the ultrasound signal may be varied and the amplitude modulated to provide oscillatory, steady state, pulsed, pulsed exponential decay, generalized periodic or randomized modulated waveforms, or a combination thereof. The supplied waveforms can display a range of characteristic timescales and amplitudes which will depend on the therapy requirements of a particular application. The ultrasound signal generation circuitry may function in any known manner to generate the desired ultrasound signal. For example, the USG may comprise one or more programmable arbitrary waveform generators (coupled to a suitable voltage and/or current amplifier to provide a signal capable of driving an ultrasound transducer as is known in the art) under control of the controller 4. Alternatively, the EFG may comprise an application specific device, such a RICH-MAR THERASOUND 3 SERIES ultrasound generator.

In the example shown in Figure 1, the USG is operable to supply an output ultrasound signal with a carrier frequency of between 0.1-2 MHz and an amplitude capable of driving the coupled transducer(s) to provide an average ultrasound energy flux (also referred to as power density) of 0.1-7 W/cm² at the treatment site. The amplitude of the carrier signal can be modulated as described above with a characteristic timescale selectable between 1 msec and 20 minutes. Depending on the required application, the USG may also supply multiple dependent or independent ultrasound signals, for example to supply the USD with multiple signals of differing phase, or to supply the USD with multiple independent signals with different waveforms (such as pulsed wave or continuous wave) and/or different times of application.

The USD is also capable of providing the controller with measurements detailing the amount of ultrasound energy deposited at the treatment site using techniques known in the art. These parameters, and any others appropriate to a particular application, can be used to form an ultrasound discharge profile. Ultrasound discharge profiles are stored by the controller 4 for subsequent analysis. Furthermore, based on the ultrasound discharge profile parameters (in addition to similar operational parameters relating to the EFG and diagnostic information derived from the DDS, described further below), the functioning can be responsively controlled by the controller 4. In other embodiments, aspects of the feedback control may be internal to the USG.

### THERAPEUTIC ULTRASOUND SIGNAL DELIVERY COMPONENT (USD)

In operation, the USD 12 serves to interface the ultrasound signals generated by the USG 10 with tissue at the treatment site and, in the example described here, the USD is interchangeably coupled to the USG such that different USD configurations can be employed as required for specific applications. The USD will generally comprise one or more ultrasound transducers for coupling ultrasound derived from the ultrasound signals supplied by the USG to the treatment site.

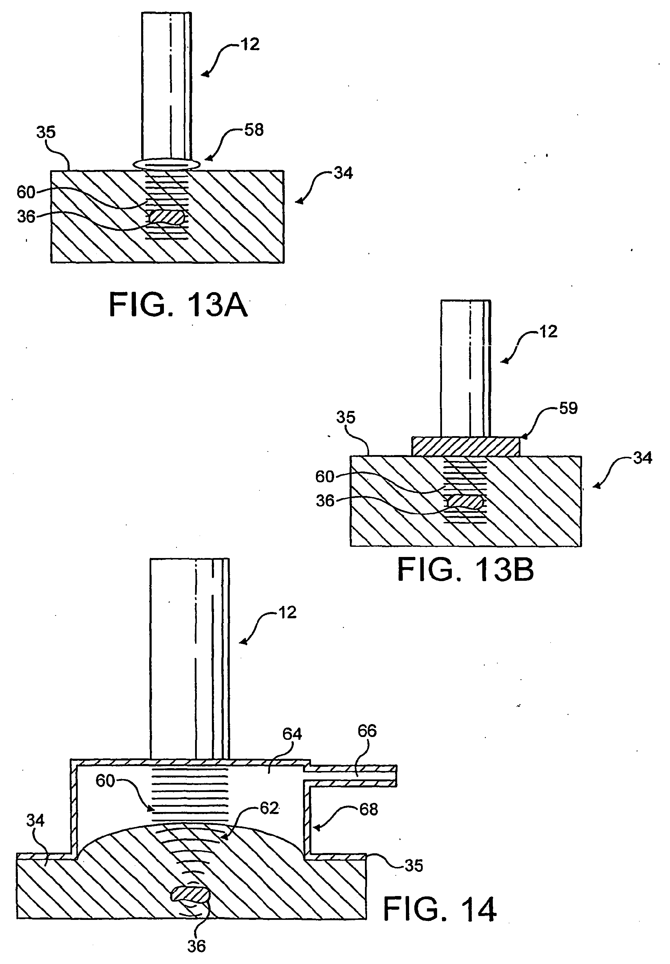

Figure 13A and Figure 13B are schematic side views showing two ways in which a USD 12 can be coupled to a tissue surface 35 to expose a treatment site 36 to ultrasound. As noted above, the USD includes an ultrasound transducer (not shown) to produce ultrasound in response to signals from the USG. In these examples, the ultrasound transducer is designed to produce a columnar beam as schematically indicated by the planar wave-fronts 60. The ultrasound power density will decay with increasing distance from the transducer in a manner while is dependent on the ultrasound absorbance characteristics of the surrounding tissue 34, the treatment site 36, and any other intervening material. Particular ultrasound frequencies may be employed to enhance ultrasound absorption at the treatment site relative to the surrounding tissue. In Figure 13A a mediating contact gel 58 facilitates acoustic coupling between the USD and the tissue surface. In Figure 13B a mediating adhesive pad 59 facilitates both mechanical and acoustic coupling between the USD and the tissue surface.

To minimize possible damage to surrounding tissue, it may be desirable to produce a focussed beam of ultrasound such that the power density is less in the surrounding tissue than at the treatment site.

Figure 14 is a schematic side view showing a USD 12 coupled to tissue surface 35 via a contact pressure vessel 68 filled containing a mediating gel 64. The contact pressure vessel is open to the tissue surface 35 but otherwise sealed such that a connection 66 to a vacuum pump (not shown) lowers the pressure of the mediating gel and causes distortion of the tissue surface within the contact pressure vessel 68, as shown in the figure.

The USD provides a columnar beam of ultrasound as above. However; the curved nature of the tissue surface provides different refraction conditions across the planar wave-fronts 60 such that the wave-fronts become curved, and hence the ultrasound beam focussed, within the tissue 34. The distorted tissue surface may therefore be seen to adopt a lens configuration to focus the ultrasound. The pressure within the contact pressure vessel 68 can be adjusted to vary the distortion of the tissue surface and so adjust the focal point of the ultrasound to coincide with the treatment site 36.

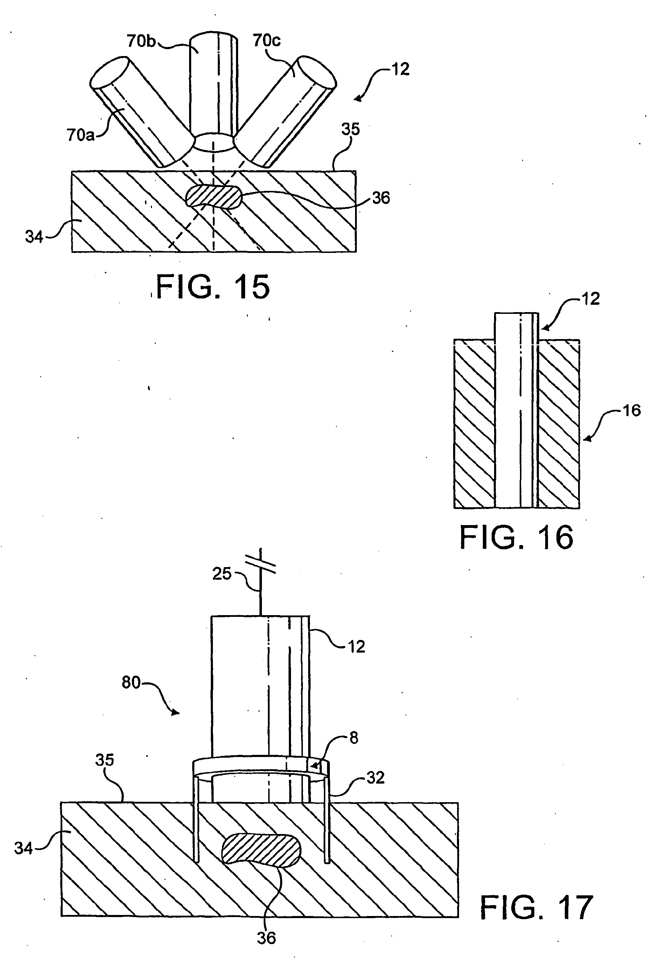

Figure 15 is a schematic perspective view of a lower portion of another example USD. In this example, the USD comprises three ultrasound transducers 70a, 70b, 70c. The ultrasound transducers provide columnar beams of ultrasound in response to ultrasound signals from the USG. Each of the three ultrasound transducers may receive the same signal, different independent signals, or a common signal but with different phase delays. The USD is held against the tissue surface 35 such that the respective beams of the ultrasound from each of the transducers converge at the treatment site 36. As with the focussing above, this allows increased power to be applied to the treatment site than to a corresponding volume of surrounding tissue 34. The individual transducers may be in contact with a mediating gel to provide a spatial buffer with good ultrasound transmission characteristics.

In addition to the example embodiment shown in Figure 15, many more configurations employing multiple ultrasound transducers could be used in a USD (and driven by an appropriately controlled USG) depending on the application. Other examples include an array of transducers mounted on a deformable concave surface to provide variable focussing, or a planar phased-array driven by the USG under control of the controller 4 to provide focussing via appropriate phasing of the ultrasound signals to individual transducers.

Whilst we have described the above USD configurations in the context of the tumour therapy device 2 shown in Figure 1B, it will be appreciated that the USD configurations described may also be used independently in other applications in which administration of ultrasound is desired. Examples include sonoporation, ultrasound diagnosis, etc. Accordingly, we disclose an ultrasound delivery component comprising an ultrasound transducer coupled to a contact pressure vessel arranged to distort a tissue surface to provide focussing of said ultrasound.

### DIAGNOSTIC ULTRASOUND SIGNAL GENERATING CIRCUITRY (DG) AND DIAGNOSTIC ULTRASOUND DELIVERY AND SCANNING COMPONENT (DDS)

The tumour therapy device 2 also includes a diagnostic ultrasound facility which, with reference to Figure 1B, comprises the DG 14 coupled to the DDS 16 by cabling 28. It will be appreciated that the diagnostic ultrasound facility is optional, and that the tissue and cell ablation abilities of the device described here do not depend on its presence.

The DG and DDS may comprise conventional units providing conventional ultrasound images of the treatment site and surrounding tissue. These images may be obtained prior to introducing either the EFD or the USD to assist guiding, or may be obtained concurrently with the EFD and/or the USD to provide continuous diagnostic information during treatment. If the diagnostic ultrasound facility is to provide information throughout the treatment process, the DDS may be combined with either a USD or EFD to form a single unit.

Figure 16 is a schematic section view of combined USD 8 and DDS 16. In this example the DDS 16 contains its own ultrasound transducer (not shown) although it is also be possible for the DDS to rely on ultrasound signals generated by the USD such that the DDS component need only contain an ultrasound scanner.

The diagnostic information provided by the DDS can be coupled to the controller 4 to allow active control of treatment parameters (e.g. delivered ultrasound power density, electric field strength, aiming, positioning, treatment duration etc.) in response to conditions at the treatment site as prescribed by the requirements of a particular therapeutic application. The information returned by the DDS can also ensure continued optimized positioning of the EFD or USD during their respective treatment phases.

For practical reasons, especially when the application requires the treatment site to be exposed to electric fields and ultrasound simultaneously, alternatively or in rapid succession, it may be preferable for the USD and EFD to be combined into a single delivery head.

Figure 17 is a schematic view showing one example of a USD and EFD combined delivery head 80 for application to a treatment site 36 located at depth beneath a tissue surface 35. A USD 12 is surrounded by an EFD 8, both of these are similar to and will be understood from the descriptions given above. Cabling 25 routes signals to and from the controller 4, the EFG 6 and the USG 10. In operation, the combined delivery head 80 is located such that the needles 32 of the EFD 8 are positioned either side of the treatment site 36 within the tissue 34 and the USD 12 is positioned against the tissue surface 35 above the treatment site. Whilst only relatively simple EFD and USD configurations are employed in this example combined delivery head 80, any of the additional features outlined above (e.g. multiple transducers, ultrasound focussing, contact pad electrodes, hollow needles, etc.) may also be included.

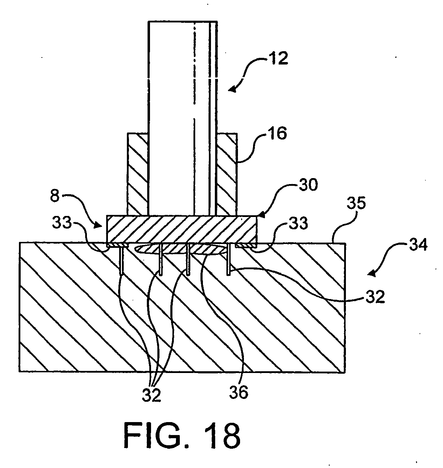

Figure 18 is a schematic view showing another example of a USD and EFD combined delivery head 80 for application to a treatment site located at a tissue surface 35. The USD 12 and the EFD 8 will be understood from the examples given above. A DDS 16 is also included and mounted on the USD in a manner which will be understood the description of Figure 16 above. The EFD in this example comprises an EFD body 30 which supports six contact electrodes (an array of four needles 32 and two conductive parts 33 of an intermediate contact pad (not shown)). In operation, the combined delivery head is located such that an electric field can be applied to the treatment site 36. As before, the electric field can be applied between any permutations of contact electrodes 32, 33 to provide the desired electric field configuration and time dependence as predetermined by an operator, or actively determined by the controller 4 in response to conditions at the treatment site. The transducer component (not shown) of the USD is separated from the tissue surface 35 by the EFD body.

### CONTROLLER

The controller controls the amplitudes, waveforms, specific signal routings for multiple contact electrodes or transducers, treatment durations etc applied by the USG and the EFG to the USD and EFD respectively by sending appropriately encoded control signals to the USG or the EFG which contain standard circuitry to allow them to respond accordingly.

By employing a centralised controller, the electric field and ultrasound components of the tumour therapy device can be co-ordinated to provide the most effective treatment. For instance, the electric field energy can be supplied at a level that is dependent on, and commensurate with, the ultrasound energy deposition and vice versa. The controller can operate to ensure that the energy deposition of the ultrasound or the electric field, or the combined energy deposition, does not exceed a pre-determined level that might otherwise cause tissue damage.

The controller also functions to apply the ultrasound and electric field in the required sequence. For instance, some therapies might require rapid alternation between ultrasound and electric field application, some might require each to applied simultaneously and some might require extended exposure to one and then the other. The controller is also operable to react to user input, diagnostic information from the DDS, or previously stored discharge profiles, to change any of the operational parameters (such as the focussing of the ultrasound, amplitude of electric field, treatment duration etc.) to provide continuously optimized therapy parameters. These functions can be performed using appropriate algorithms designed according to specific therapy requirements.

The controller 4 is operable to facilitate manual and/or automated electric field generation by the EFG (and subsequent delivery through the EFD) and ultrasound generation by the USG (and subsequent delivery through the USD). As noted above, the controller can modify the USG and/or EFG operation based upon on previously recorded, or currently sampled, discharge profiles and/or diagnostic output from the DDS.

The controller 4 can also display parameters regarding the both the EFG operation (such as set voltage, delivered voltage, predicted electric field, current density in the vicinity of the EFD, energy deposition at the treatment site, treatment duration, impedance at the treatment site, internal circuitry resistance, etc. or combinations thereof) and parameters regarding the USG operation (such as power density output, predicted power density at the treatment site, distance to the treatment site, treatment duration, modulation frequency, ultrasound frequency etc. or combinations thereof) to assist an operator in overseeing the therapy. These parameters can be determined using standard techniques. The controller is arranged to modify the EFG or USD operation in response to any of these parameters as appropriate for particular therapy requirements and applications.

The apparatus having been described in detail, we now provide a general description of the processes of sensitisation, electrosensitisation, disruption, as well as description of ultrasound and cell-death facilitating agents, in order that the cell ablation process as effected by the apparatus, and the conditions under which this may be achieved and optimised, be understood further. Knowledge of such processes, as provided below, enables the apparatus as described to be used in the most efficient manner.

### SENSITISATION

According to a general aspect, cells are sensitised to render them more susceptible to disruption by a stimulus than unsensitised cells. Accordingly, "sensitised" cells are those that have been treated in order to render them more susceptible to disruption by exposure to a stimulus than an unsensitised cell. Such cells are capable of being disrupted at a target site by exposure to a stimulus. Thus, we provide in general a means of disrupting a cell by exposure to sensitiser followed by exposure to a disrupter. Various combinations of sensitisers and disrupters may be employed.

For example, sensitisation may be achieved by exposing the cells to ultrasound. Preferably, such ultrasound sensitised cells are capable of being disrupted by subsequent exposure to an electric field. This aspect is exemplified in Example 11.

However, a preferred means of sensitisation is electrosensitisation. Electrosensitisation is described in our International Patent Application Number PCT/GB00/02848 (published as WO/01/07011), and is described in detail below. The disruption stimuli include laser light and other energy sources, but in a highly preferred embodiment comprises ultrasound.

In the particular apparatus described here, exposure of a cell to an electric field, and/or ultrasound generated by the device causes the cell to be sensitised. Preferably, the apparatus is used in such a way that it provides an electric field to sensitise the cell.

### ELECTROSENSITISATION

The term "electrosensitisation" encompasses the destabilisation of cells, such that they are more sensitive to disruption by a stimulus (for example, ultrasound) than otherwise. Thus, the apparatus as described here is operable to effect electrosensitisation of a cell.

In electrosensitisation, exposure of a cell to an electric field results in membrane destabilisation and sensitisation of the cell to further stimulus. The cell may be subject to a momentary exposure, or prolonged exposure to electric field. The electric field may be applied in the form of one or more pulses. Alternatively, the cells are exposed to a constantly present field, which may vary in strength, intensity, direction, etc. The strength of the electric field may be adjusted up or down depending upon the resilience or fragility of cells in the targeted tissue. The apparatus as described here is capable of being operated in different ways to deliver such electric fields.

Electrosensitisation typically occurs in the absence of an agent to be loaded into the cell. Electroporation, which facilitates passage of agents into the cell, occurs in the presence of an exogenous agent to be loaded, and is well known in the art. As noted above, other agents which facilitate cell death may be applied to the cell to promote cell death; however, these agents are typically not present when electrosensitisation takes place.

As used herein, "electric field energy" is the electrical energy to which a cell is exposed during an electrosensitisation procedure as described herein. Preferably the electric field has a strength of from about 1 Volt/cm to about 10 kVolts/cm or more under *in vivo* conditions (see WO97/49450), and the apparatus is operatively capable of delivering such field strengths at least.

As used herein, the term "electric field" includes one or more pulses at variable capacitance and voltage and including exponential and/or square wave and/or modulated wave and/or modulated square wave forms, and the apparatus is capable of delivering any and all of these wave forms. References to electric fields and electricity should be taken to include reference the presence of an electric potential difference in the environment of a cell. Thus, in the apparatus described here, the environment is set up by maintaining a potential difference between two or more electrodes. Such an environment may be set up by way of static electricity, alternating current (AC), direct current (DC), etc, as known in the art. The electric field may be uniform, non-uniform or otherwise, and may vary in strength and/or direction in a time dependent manner.

Electroporation has been used in both *in vitro* and *in vivo* procedures to introduce foreign material into living cells. With *in vitro* applications, a sample of live cells is first mixed with the agent of interest and placed between electrodes such as parallel plates. Then, the electrodes apply an electrical field to the cell/implant mixture. Examples of systems that perform *in vitro* electroporation include the Electro Cell Manipulator ECM600 product, and the Electro Square Porator T820, both made by the BTX Division of Genetronics, Inc (see US Patent No 5869326).

The known electroporation techniques (both *in vitro* and *in vivo*) function by applying a brief high voltage pulse to electrodes positioned around the treatment region. The electric field generated between the electrodes causes the cell membranes to temporarily become porous, whereupon molecules of the agent of interest enter the cells. In known electroporation applications, this electric field comprises a single square wave pulse on the order of 1000 V/cm, of about 100 µs duration. Such a pulse may be generated, for example, in known applications of the Electro Square Porator T820.

Electrosensitisation may be performed in a manner substantially identical to the procedure followed for electroporation, with the exception that the electric field is delivered in the absence of an exogenous agent of interest, as set forth below, and may be carried out at different electric field strengths (and other parameters) from those required for electroporation. For example, lower field strengths may be used for electrosensitisation. Thus, systems for electroporation may be used for delivery of electric fields to cells, tissues, etc, in the methods and compositions described here.

Preferably, the electric field has a strength of from about 1V/cm to about 10 kV/cm under *in vitro* conditions. Thus, the electric field may have a strength of 1 V/cm, 2 V/cm, 3 V/cm, 4 V/cm, 5 V/cm, 6 V/cm, 7 V/cm, 8 V/cm, 9 V/cm, 10 V/cm, 20 V/cm, 50 V/cm, 100 V/cm, 200V/cm, 300 V/cm, 400 V/cm, 500 V/cm, 600 V/cm, 700 V/cm, 800 V/cm, 900 V/cm, 1 kV/cm, 2 kV/cm, 5 kV/cm, 10 kV/cm, 20 kV/cm, 50 kV/cm or more. More preferably from about 0.5 kV/cm to about 4.0 kV/cm under *in vitro* conditions. Preferably the electric field has a strength of from about 1 V/cm to about 10 kV/cm under *in vivo* conditions. However, the electric field strengths may be lowered where the number of pulses delivered to the target site are increased. Thus, pulsatile delivery of electric fields at lower field strengths is envisaged.

Preferably the application of the electric field is in the form of multiple pulses such as double pulses of the same strength and capacitance or sequential pulses of varying strength and/or capacitance. As used herein, the term "pulse" includes one or more electric pulses at variable capacitance and voltage and including exponential and/or square wave and/or modulated wave/square wave forms.

Preferably the electric pulse is delivered as a waveform selected from an exponential wave form, a square wave form, a modulated wave form and a modulated square wave form.

A preferred embodiment employs direct current at low voltage. Thus; we disclose the use of an electric field which is applied to the cell, tissue or tissue mass at a field strength of between 1 V/cm and 20V/cm, for a period of 100 milliseconds or more, preferably 15 minutes or more.

The use of electric fields for disruption sensitised cells may in general employ the same parameters as those set out above for electricity as a sensitiser.

### ULTRASOUND

According to one aspect, cells which have been sensitised (in particular, electrosensitised) may be disrupted by the application of ultrasound directed at a target tissue and/or cell. According to another aspect, ultrasound may be used as a means of sensitising a cell, i.e., a sensitiser. The apparatus as described here is capable of delivering ultrasound to a cell to enable either sensitisation or disruption of the cell.

As used herein, the term "ultrasound" refers to a form of energy which consists of mechanical vibrations the frequencies of which are so high they are above the range of human hearing. Lower frequency limit of the ultrasonic spectrum may generally be taken as about 20kHz. Most diagnostic applications of ultrasound employ frequencies in the range 1 and 15MHz' (From Ultrasonics in Clinical Diagnosis, P.N.T. Wells, ed., 2nd. Edition, Publ. Churchill Livingstone [Edinburgh, London & NY, 1977]).

Ultrasound has been used in both diagnostic and therapeutic applications. When used as a diagnostic tool ("diagnostic ultrasound"), ultrasound is typically used in an energy density range of up to about 100 mW/cm² (FDA recommendation), although energy densities of up to 750mW/cm² have been used. In physiotherapy, ultrasound is typically used as an energy source in a range up to about 3 to 4 W/cm² (WHO recommendation). In other therapeutic applications, higher intensities of ultrasound may be employed, for example, HIFU at 100W/cm² up to 1kW/cm² (or even higher) for short periods of time. The term "ultrasound" as used in this specification is intended to encompass diagnostic, therapeutic and focused ultrasound.

Focused ultrasound (FUS) allows thermal energy to be delivered without an invasive probe (see Morocz et al 1998 Journal of Magnetic Resonance Imaging Vol.8, No.1, pp.136-142. Another form of focused ultrasound is high intensity focused ultrasound (HIFU) which is reviewed by Moussatov et al in Ultrasonics (1998) Vol.36, No.8, pp.893-900 and TranHuuHue et al in Acustica (1997) Vol.83, No.6, pp.1103-1106.

Preferably, a combination of diagnostic ultrasound and a therapeutic ultrasound is employed. This combination is not intended to be limiting, however, and the skilled reader will appreciate that any variety of combinations of ultrasound may be used. Additionally, the energy density, frequency of ultrasound, and period of exposure may be varied. What is important is that the application of ultrasound is able to disrupt the sensitised target cells, or as the case may be, to sensitise them to disruption by application of a stimulus.

Preferably the ultrasound is applied to target tissue with sufficient strength to disrupt sensitised cells but without damaging the surrounding tissues. As used herein, "disrupt" or "ablate" signifies that the target tissue and/or the cells thereof is or are damaged, for example by lysis, necrosis, apoptosis, etc. Preferably, the damage is such that the cells are either killed outright or recognised as damaged by the internal defence systems of the organism and eliminated thereby. Preferably, a tissue or cell is "ablated" when sufficient damage takes place that it is eliminated from the body of the organism.

Preferably the exposure to an ultrasound energy source is at a power density of from about 0.05 to about 100 Wcm⁻². Even more preferably, the exposure to an ultrasound energy source is at a power density of from about 1 to about 15 Wcm⁻².

Preferably the exposure to an ultrasound energy source is at a frequency of from about 0.015 to about 10.0 MHz. More preferably the exposure to an ultrasound energy source is at a frequency of from about 0.02 to about 5.0 MHz or about 6.0 MHz. Most preferably, the ultrasound is applied at a frequency of 3 MHz.

Preferably the exposure is for periods of from about 10 milliseconds to about 60 minutes. Preferably the exposure is for periods of from about 1 second to about 5 minutes. More preferably, the ultrasound is applied for about 2 minutes. Depending on the particular target cell to be disrupted, however, the exposure may be for a longer duration, for example, for 15 minutes.

Advantageously, the target tissue is exposed to an ultrasound energy source at an acoustic power density of from about 0.05Wcm⁻² to about 10Wcm⁻² with a frequency ranging from about 0.015 to about 10MHz (see WO 98/52609). However, alternatives are also possible, for example, exposure to an ultrasound energy source at an acoustic power density of above 100Wcm⁻², but for reduced periods of time, for example, 1000Wcm⁻² for periods in the millisecond range or less.

Preferably the application of the ultrasound is in the form of multiple pulses; thus, both continuous wave and pulsed wave (pulsatile delivery of ultrasound) may be employed in any combination. For example, continuous wave ultrasound may be applied, followed by pulsed wave ultrasound, or vice versa. This may be repeated any number of times, in any order and combination. The pulsed wave ultrasound may be applied against a background of continuous wave ultrasound, and any number of pulses may be used in any number of groups.

Preferably, the ultrasound comprises pulsed wave ultrasound. In a highly preferred embodiment, the ultrasound is applied at a power density of 0.7 Wcm⁻² or 1.25 Wcm⁻² as a continuous wave. Higher power densities may be employed if pulsed wave ultrasound is used.

Use of ultrasound is advantageous as, like light, it can be focused accurately on a target. Moreover, ultrasound is advantageous as it can be focussed more deeply into tissues unlike light. It is therefore better suited to whole-tissue penetration (such as but not limited to a lobe of the liver) or whole organ (such as but not limited to the entire liver or an entire muscle, such as the heart) therapy. Another important advantage is that ultrasound is a non-invasive stimulus which is used in a wide variety of diagnostic and therapeutic applications. By way of example, ultrasound is well known in medical imaging techniques and, additionally, in orthopaedic therapy. Furthermore, instruments suitable for the application of ultrasound to a subject vertebrate are widely available and their use is well known in the art.

### LOW INTENSITY SENSITISATION AND DISRUPTION

As noted above, low intensity electric fields may be employed to sensitise cells. Low voltage strengths may be set up using alternating current or preferably using direct current (DC). Where direct current is used, it may be applied in either a pulsed or continuous manner, and likewise where alternating current is used. Such cells may preferably be disrupted with low intensity ultrasound.

Thus, in a particular embodiment, direct current at low voltage is used to electrosensitise cells. Electric field strengths as low as from 1V/cm, preferably 5V/cm to 100V/cm more, preferably between 10V/cm and 20V/cm, e.g., 1, 2, 3, 4, 5, 6, 7, 8, 9, 10, 11, 12, 13, 14, 15, 16, 17, 18, 19 or 20V/cm or more may be employed. Electric fields may also be measured in terms of current; preferably, the current applied is between 100µA to 200mA, preferably between 1mA and 10mA. Thus, the current applied may be about 100µA, about 200µA, about 300µA, about 400µA, about 500µA, about 600µA, about 700µA, about 800µA, about 900µA, about 1mA, about 2mA, about 3mA, about 4mA, about 5mA, about 6mA, about 7 mA, about 8 mA, about 9 mA, about 10 mA, about 20mA, about 50mA, about 100mA, about 200 mA, or more.

Where low intensity fields and/or direct current is used, the time of exposure of the cells to the field may typically be in the order of seconds to minutes. Thus, the cells may be exposed to the electric field for more than 100µs, for example, 1 millisecond or more, preferably 0.5 seconds or more. Most preferably, the cells are exposed for more than 1 second, preferably, 5, 10, 30, 60, 120, 180, 240, 300 or more seconds. The cells may be exposed for 1, 2, 3, 4, 5, 6, 7, 8, 9, 10, 15, 20, 25, 30 or more minutes.

The characteristics of the electric field may be constant or vary over the course of exposure, for example, the strength and/or direction of the electric field may be varied. The electric field strength may be steady during the exposure, or may vary in intensity. For example, where the cell is exposed to the field under a constant current setting, the electric field strength may vary. Thus, for example, where a 5mA constant current is being applied, the electric field strength may vary between 10V/cm and 20V/cm.

In a highly preferred embodiment, the electric field is applied to the cell, tissue or tissue mass at a field strength of between 10V/cm and 20V/cm. The electric field is applied preferably for a period of 100 milliseconds or more, preferably 15 minutes or more. The electric field may be applied continuously or in a pulsed manner.

Treatment of tumours with low voltage/current direct current (DC) without application of ultrasound ("electrochemical treatment") has been described in Nordenstrom, Am. J. Clin. Oncol. 1989, 12, 530-536 and Wojcicki et al., Med. Sci. Monit. 2000, 6, 498-502. However, in all cases, both in the clinic and in animal models it has been found that such treatment leads to the formation of necrotic lesions and in many cases the target tissues re-establish at the treatment site. The low voltage electrosensitisation described here may however usefully employ the conditions and techniques described in these two documents.

Cells treated with low electric field strengths, for example, using DC, may be disrupted using ultrasound or other stimulus. Such electrosensitised cells may be disrupted by the use of low intensity ultrasound, e.g., in the diagnostic and/or therapeutic ranges: 100 mW/cm² up to 750mW/cm² or in a range up to about 3 to 4 W/cm² or more, as described in further detail below.

Such treatment with low intensity electric fields, preferably coupled with low intensity ultrasound may be employed for the treatment of benign disorders and also in the cosmetics industry. Benign disorders which may be treated include any disorder which may be cured, treated or addressed by cell or tissue disruption or excision. For example, low intensity ultrasound and/or electric fields may be used to treat skin conditions such as warts, papilomas, psoriasis, eczema, moles, etc. In addition, therapies employing this aspect can be used to accomplish drug- and surgery-free treatment of benign disorders such as benign breast and prostate disease, human papilloma virus (HPV) infection (condylomata acuminata, Longstaff & von Krogh, 2001, Reg. Tox. Pharm. 33, 117-137), eradicating benign granulomatous tissues remaining after localised infections (Hildebrandt et al.,1998, Strahlenther Onkol., 174. 580-588).

Lipomas, which are fatty deposits, as well as other conditions involving deposits of excess fat, may be also treated. Thus, our methods may be used to destroy or disrupt adipose cells or tissue as a substitute for cosmetic treatments such as liposuction.

Furthermore, our methods are suitable for treatment of relatively large areas or parts of the body, particularly for removal of large areas of adipose tissues for cosmetic purposes.

### AGENTS WHICH FACILITATE CELL DEATH

According to a highly preferred embodiment, the target cell, tissue or tissue mass is further exposed to an agent which is capable of facilitating cell death. Thus, the apparatus in a preferred embodiment comprises means for delivering a cell-death facilitating agent to the cell, or its vicinity. Such cell-death facilitating agent delivery means may comprise one or more hollow needles in fluid communication with a reservoir, as described in detail above.

An agent which is capable of facilitating cell death is one which, when exposed to a target cell, tissue or tissue mass, is necessary or sufficient to cause cell death. Preferably, such an agent is one which enhances the cell killing ability of treatment with sensitisation and disruption (e.g., electric field/ultrasound administration). Contact with such an agent therefore preferably enhances the cell killing, disruption or ablation effected by sensitisation and disruption, for example, exposure to electric field and ultrasound in any order. In a preferred embodiment, cell-death facilitating agents are used to "mop up" and destroy any cells which have, whether inadvertently or on purpose, been unaffected by the treatment, e.g., application of electric field and ultrasound.

Such facilitating agents may have the ability to promote cell death on their own. Indeed, any agent used for treatment of tumours or cancers as known in the art is a suitable candidate for use as a cell-death facilitating agent. However, the term "agent which facilitates cell death" and "cell death facilitating agent" should be taken to include those agents which work to promote cell death when used in combination with cell sensitisation (for example electrosensitisation) and disruption, for example, ultrasound exposure, as set out above. Preferably, the administration of such cell death facilitating agents enhances cell killing by more than 10%, more than 20%, more than 30%, more than 40%, more than 50%, more than more than 60%, more than 70%, more than 80%, more than 90%, more than 100% compared to the cell killing efficiency of the treatments (e.g., ultrasound/electric field) alone.

The cell-death facilitating agent may work in any number of ways. For example, the agent may be directly toxic to the cell. Agents in this category include cytotoxics, which are used in tumour therapy. The agent may further be one which causes an immune reaction in the host, to the effect that the target cell, tissue, etc is eliminated or killed by the patient's normal immune processes (including both cell-mediated and humoural immune responses). Examples of such agents include cytotoxic T cells and dendritic cells. Furthermore, the agent may comprise an agent which is capable of recruiting immune responses, such as a cytokine. For example, cytokines such as IL-2, GMCSF etc may be used to promote the host's immune response.

The cell-death facilitating agent may be applied directly to the cell, or in its vicinity. It will be appreciated that, where the cell-death facilitating agent is proteinaceous in nature (e.g., a peptide, a polypeptide, a protein, or a fragment of any of these), the cell-death facilitating agent may be administered in the form of a nucleic acid encoding the peptide, polypeptide, etc. Such a nucleic acid may preferably be in the form of an expression vector, and methods for making such vectors and constructs, and the induction of expression from these, are known in the art. Furthermore, we envisage the use of cell-death facilitating agents in the form of cells producing such agents, for example, a cell capable of expressing a cell-death facilitating agent by virtue of comprising a nucleic acid sequence encoding that agent. The cell may be transfected or transformed with a nucleic acid, for example, an expression vector, encoding the cell death facilitating agent, for example, cytotoxic agent, a cytostatic agent, a cytokine, GM-CSF, IL-2 or an immunogen.

The cell-death facilitating agent need not necessarily be molecular in nature. Thus, the use of treatments which cause cell killing by other means, as known in the art, is also encompassed. Examples include the use of radiation, whether applied externally or internally, to kill cells such as tumour cells. Methods of using radiotherapy as primary or auxiliary therapy for tumours is known in the art.

The target cell, tissue or tissue mass may be exposed to the cell-death facilitating agent either before, during or after the stimulus which disrupts the cells, e.g., ultrasound treatment where cells are electrosensitised and then exposed to ultrasound. However, in all cases, the sensitiser (for example, the electric field which electrosensitises the cell, etc) is applied substantially prior to administration of the agent. In other words, the sensitiser is applied to the target in the absence of such a cell death facilitating agent; in particular, where electrosensitisation is employed, the electric field is applied to the target in the absence of a cell death facilitating agent Thus, the cell death facilitating agent is not primarily responsible for inducing cell disruption or death, but rather acts in a supplementary role to promote cell death of cells, tissues, etc exposed to the sensitiser and disrupter (e.g., electric field and ultrasound).

The cell-death facilitating agent may be exposed to the target cell, tissue or tissue mass by any suitable manner. For example, the agent may be topically applied to the skin; this is advantageous if for example the target cell is epidermal (for example, a skin tumour). Furthermore, the agent may be systemically administered to the patient or to the system of which the target cell, etc forms a part. The agent may be administered orally (taken by mouth), nasally, delivered using liposome technology, etc. The agent may be directly injected into the tumour mass, or at or near the tumour site. The agent may be delivered in the form of a cell expressing the agent, for example a cell expressing a cytotoxic agent. Use of cells expressing such cytotoxic agents, for example, IL-2, is known in the art, and described in for example, Mir et al., J. Immunotherapy, 17, 30-38 and Orlowski, et al., 1998, Anticancer Drugs 9, 551-556.

The agent may be delivered by being loaded into a suitable carrier, such as a red blood cell carrier. Loading and delivery using red blood cells is disclosed in detail in our International Patent Application numbers PCT/GB00/02848 (published as WO/01/07011) and PCT/GB00/03056. The agent may be delivered into an intracellular compartment by the use of Membrane Translocation Sequences (MTS), as described elsewhere in this document. The agent may also suitably be administered in the form of a pharmaceutical composition, as described in further detail below.

Agents useful in the methods and compositions described here are set out below. Preferred agents which are capable of facilitating cell death include cytokines and cytotoxics, as well as nucleic acids encoding these. These are discussed in further detail elsewhere in this document.

As used herein, the term "agent" includes but is not limited to an atom or molecule, wherein a molecule may be inorganic or organic, a biological effector molecule and/or a nucleic acid encoding an agent such as a biological effector molecule, a protein, a polypeptide, a peptide, a nucleic acid, a peptide nucleic acid (PNA), a virus-like particle, a nucleotide, a deoxyribonucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a fatty acid and a carbohydrate. An agent may be in solution or in suspension (e.g., in crystalline, colloidal or other particulate form). The agent may be in the form of a monomer, dimer, oligomer, etc, or otherwise in a complex. The agent may be coated with one or more molecules, preferably macromolecules, most preferably polymers such as PEG (polyethylene glycol). Use of a PEGylated agent increases the circulating lifetime of the agent once released.

The cell death facilitating agent may be radioactive, i.e., a radionuclide which is used in radiotherapy. The radionuclide may be a radio-isotope as known in the art, for example cobalt-60, iodine-131, etc, or a molecule such as a nucleic acid, polypeptide, or other molecule as explained below conjugated with such a radio-isotope. As noted above, external radiation sources utilising such radionuclides, in the form of external and/or internal radiotherapy may also be used to facilitate cell death in the treated target cell or tissue.

It will be appreciated that it is not necessary for a single agent to be used, and that it is possible to utilise two or more cell death facilitating agents, sequentially or simultaneously, to achieve cell death. Accordingly, the term "agent" also includes mixtures, fusions, combinations and conjugates, of atoms, molecules etc as disclosed herein. For example, an agent may include but is not limited to: a nucleic acid combined with a polypeptide; two or more polypeptides conjugated to each other; a protein conjugated to a biologically active molecule (which may be a small molecule such as a prodrug); or a combination of a biologically active molecule with an imaging agent.

As used herein, the term "biological effector molecule" or "biologically active molecule" refers to an agent that has activity in a biological system, including, but not limited to, a protein, polypeptide or peptide including, but not limited to, a structural protein, an enzyme, a cytokine (such as an interferon and/or an interleukin) an antibiotic, a polyclonal or monoclonal antibody, or an effective part thereof, such as an Fv fragment, which antibody or part thereof may be natural, synthetic or humanised, a peptide hormone, a receptor, and a signalling molecule. Included within the term "immunoglobulin" are intact immunoglobulins as well as antibody fragments such as Fv, a single chain Fv (scFv), a Fab or a F(ab')₂.

Preferred immunoglobulins, antibodies, Fv fragments, etc are those which are capable of binding to antigens in an intracellular environment, known as "intrabodies" or "intracellular antibodies". An "intracellular antibody" or an "intrabody" is an antibody which is capable of binding to its target or cognate antigen within the environment of a cell, or in an environment which mimics an environment within the cell.

Selection methods for directly identifying such "intrabodies" have been proposed, such as an *in vivo* two-hybrid system for selecting antibodies with binding capability inside mammalian cells. Such methods are described in International Patent Application number PCT/GB00/00876, hereby incorporated by reference. Techniques for producing intracellular antibodies, such as anti-β-galactosidase scFvs, have also been described in Martineau, et al., 1998, J Mol Biol 280, 117-127 and Visintin, et al., 1999, Proc. Natl. Acad Sci. USA 96, 11723-11728.

An agent may include a nucleic acid, as defined below, including, but not limited to, an oligonucleotide or modified oligonucleotide, an antisense oligonucleotide or modified antisense oligonucleotide, cDNA, genomic DNA, an artificial or natural chromosome (e.g. a yeast artificial chromosome) or a part thereof, RNA, including mRNA, tRNA, rRNA or a ribozyme, or a peptide nucleic acid (PNA); virus-like particles; a nucleotide or ribonucleotide or synthetic analogue thereof, which may be modified or unmodified; an amino acid or analogue thereof, which may be modified or unmodified; a non-peptide (e.g., steroid) hormone; a proteoglycan; a lipid; or a carbohydrate. If the biological effector molecule is a polypeptide, it may be applied directly to the target area; alternatively, a nucleic acid molecule bearing a sequence encoding the polypeptide, which sequence is operatively linked to transcriptional and translational regulatory elements active in a cell at the target site, may be used. Small molecules, including inorganic and organic chemicals, are also of use. In a particularly preferred embodiment, the biologically active molecule is a pharmaceutically active agent, for example, an isotope.

A preferred embodiment comprises use of a ribozyme or an oligonucleotide such as an antisense oligonucleotide and exposing this to a target cell or tissue to facilitate cell death.

Particularly useful classes of biological effector molecules include, but are not limited to, antibiotics, anti-inflammatory drugs, angiogenic or vasoactive agents, growth factors and cytotoxic agents (e.g., tumour suppressers). Cytotoxic agents of use include, but are not limited to, diptheria toxin, Pseudomonas exotoxin, cholera toxin, pertussis toxin, and the prodrugs peptidyl-p-phenylenediamine-mustard, benzoic acid mustard glutamates, ganciclovir, 6-methoxypurine arabinonucleoside (araM), 5-fluorocytosine, glucose, hypoxanthine, methotrexate-alanine, N-[4-(a-D-galactopyranosyl) benyloxycarbonyl]-daunorubicin, amygdalin, azobenzene mustards, glutamyl p-phenylenediamine mustard, phenolmustard-glucuronide, epirubicin-glucuronide, vinca-cephalosporin,phenylenediamine mustard-cephalosporin, nitrogen-mustard-cephalosporin, phenolmustard phosphate, doxorubicin phosphate, mitomycin phosphate, etoposide phosphate, palytoxin-4-hydroxyphenyl-acetamide, doxorubicin-phenoxyacetamide, melphalan-phenoxyacetamide, cyclophosphamide, ifosfamide or analogues thereof. If a prodrug is applied to the target cell, tissue or tissue mass in inactive form, a second biological effector molecule may be applied. Such a second biological effector molecule is usefully an activating polypeptide which converts the inactive prodrug to active drug form, and which activating polypeptide is selected from the group that includes, but is not limited to, viral thymidine kinase (encoded by Genbank Accession No. J02224), carboxypeptidase A (encoded by Genbank Accession No. M27717), α-galactosidase (encoded by Genbank Accession No. M13571), ß-glucuronidase (encoded by Genbank Accession No. M15182), alkaline phosphatase (encoded by Genbank Accession No. J03252 J03512), or cytochrome P-450 (encoded by Genbank Accession No. D00003 N00003), plasmin, carboxypeptidase G2, cytosine deaminase, glucose oxidase, xanthine oxidase, β-glucosidase, azoreductase, t-gutamyl transferase, β-lactamase, or penicillin amidase. Either the polypeptide or the gene encoding it may be administered; if the latter, both the prodrug and the activating polypeptide may be encoded by genes on the same recombinant nucleic acid construct.

Preferably the biological effector molecule is selected from the group consisting of a protein, a polypeptide, a peptide, a nucleic acid, a virus-like particle, a nucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid and a carbohydrate or a combination thereof (e.g., chromosomal material comprising both protein and DNA components or a pair or set of effectors, wherein one or more convert another to active form, for example catalytically).

The biological effector molecule is preferably an immunomodulatory agent or other biological response modifier. Also included are polynucleotides which encode metabolic enzymes and proteins, including antiangiogenesis compounds, e.g., Factor VIII or Factor IX.

### CYTOTOXICS

The cell-death facilitating agents described above may be used, whether alone or in combination with each other, together with ultrasound/electric field treatment as delivered by the apparatus described here. Preferred cell-death facilitating agents which are delivered in a preferred embodiment of the apparatus include cytotoxics and cytokines.

"Cytotoxicity" refers to the cell killing property of a chemical compound (such as a food, cosmetic, or pharmaceutical) or a mediator cell (cytotoxic T cell). In contrast to necrosis and apoptosis, the term cytotoxicity need not necessarily indicate a specific cellular death mechanism. For example, cell mediated cytotoxicity (that is, cell death mediated by either cytotoxic T lymphocytes [CTL] or natural killer [NK] cells) combines some aspects of both necrosis and apoptosis. The terms "cytotoxic" and "cytoxic drug" are used interchangeably, and refer to any of a group of drugs that are toxic to cells and cause cell death or prevent any cell process such as cell growth, proliferation, or replication. The latter are also referred to as "cytostats" or "cytostatic drugs".

Preferably, the cytotoxic comprises chemotherapeutic agents having an antitumor effect. Cytotoxics are used mainly to treat cancer, although some have other uses (such as for treatment of other disorders, such as psoriasis and rheumatoid arthritis). Cancer treatment with cytotoxics is known as chemotherapy and has a variety of purposes. The cytotoxics may be used to shrink a tumour before surgery (neoadjuvant chemotherapy); they may be used after the primary tumour has been treated with surgery or radiotherapy to prevent the spread and growth of secondary tumours (adjuvant chemotherapy), or they may be the main treatment for the disease. Chemotherapy may be given to cure the disease or, if cure is not possible, to control its symptoms (palliative chemotherapy).

Cytotoxics suitable for use for preferred embodiments include alkylating drugs, antimetabolites, vinca alkaloids, cytotoxic antibiotics, platinum compounds (e.g. carboplatin), taxanes, topoisomerase inhibitors, procarbazine, crisantaspase, hydroxyurea, Rituximab (a monoclonal antibody) and aldesleukin (an interleukin). Other preferred examples of cytotoxics include bleomycin, neocarcinostatin, suramin, doxorubicin, carboplatin, taxol, mitomycin C, cisplatin, Azathioprine, (Imuran), Cyclophosphamide, (Cytoxan), Methotrexate (Rheumatrex), as well as other cytotoxic drugs related to cyclophosphamide (Cytoxan) including chlorambucil (Leukeran) and nitrogen mustard (Mustargen).

Sex hormones have been used to treat cancer, and may also be used. Tumours of the prostate gland are often stimulated by male sex hormones (the androgens) and so these cancers may be treated with oestrogens (to oppose the androgens) or with anti-androgens. Analogues of gonadorelin, such as buserelin, goserelin, leuprorelin, and triptorelin, may also be used. Some breast cancers are stimulated by oestrogens; such cancers respond to the oestrogen antagonists tamoxifen and toremifene or to aromatase inhibitors. Any of the above cytotoxics may be employed in the preferred methods described here.

Also included within the term "cytotoxic" are cells such as Cytotoxic T lymphocytes (CTL) and Natural Killer (NK) cells. Furthermore, it has been found that compounds which inhibit the effects of VEGF, such as PTK787/ZK 222584, have the potential to provide effective and well-tolerated therapies for the treatment of solid tumours (Wood JM, 2000, Medicina (B Aires) 60 Suppl 2:41-7). Accordingly, the use of such compounds as cell-death facilitating agents is also envisaged.

The cytotoxic may be taken by mouth or given by injection or infusion. In general, cytotoxics may be administered in any suitable manner. Preferred routes of administration include administration systemically, orally and nasally. A highly preferred route is local administration to the target tissue. Any suitable formulation, as disclosed in further detail below, may be employed. A combination of two, three, or more cytotoxics, optionally together with one, two, three or more cytokines (as disclosed in further detail elsewhere) may be given. The effects of cytotoxics may need to be carefully monitored and blood tests carried out regularly.

### CYTOKINES

In a further embodiment, the cell-death facilitating agent which is delivered in a preferred embodiment of the apparatus comprises an agent which is capable of stimulating, reinforcing, recruiting, or boosting an immune response of the patient. Preferably such an immune response is directed against a cell in the target site, preferably, a sensitised cell. More preferably, the cell is an electrosensitised cell which has been exposed to ultrasound, or a ultrasound sensitised cell which has been exposed to an electric field, for example, by means of the apparatus described here. Most preferably, the cell is a disrupted or ablated cell which has been so exposed. In a highly preferred embodiment, administration of a cell-death facilitating agent results in a cell being destroyed by means of one or more components of the patient's immune system.

Such agents preferably stimulate host immune responses such as recruitment of killer cells such as cytotoxic T-cells and dendritic cells to the target site. For example, immunogens or antigens may be administered to the patient as part of the therapy described here, to enhance cell killing.

The term "cytokine" may be used to refer to any of a number of soluble molecules (e.g., glycoproteins) released by cells of the immune system, which act nonenzymatically through specific receptors to regulate immune responses. Cytokines resemble hormones in that they act at low concentrations bound with high affinity to a specific receptor. Preferably, the term "cytokine" refers to a diverse group of soluble proteins and peptides which act as humoral regulators at nano- to picomolar concentrations and which, either under normal or pathological conditions, modulate the functional activities of individual cells and tissues.

Particular examples of cytokines which are suitable for use in the methods and compositions described include interleukins, lymphokine, interferon, Colony Stimulating Factors (CSFs) such as Granulocyte-Colony Stimulating Factor (G-CSF), Macrophage-Colony stimulating factor (M-CSF) and Granulocyte-Macrophage-Colony stimulating factor (GM-CSF), GSF, Platelet-Activating Factors (PAF), Tumor Necrosis Factor (TNF).

Thus, interleukins such as IL1, IL2 and IL4, as well as interferons such as IFN-α, IFN-β and IFN-γ may be used in the methods described here. Tumour necrosis factors TNF-α (cachetin), TNF-β (lymphotoxin) may also be suitably employed.

Preferred cytokines are those which are capable of recruiting immune responses, for example, stimulation of dendritic cell or cytotoxic T cell activity, or which are capable of recruiting macrophages to the target site. In a highly preferred embodiment, the cytokine comprises IL-2, GM-CSF or GSF.

### APOPTOSIS

Cell death can occur by either of two distinct mechanisms, necrosis or apoptosis. In addition, certain chemical compounds and cells are said to be cytotoxic to the cell, that is, to cause its death. According to a preferred aspect, exposure of cells to a sensitiser and a disrupter (e.g., electric field and ultrasound in any order) delivered by means of the described apparatus results in at least a proportion of cells undergoing cell death by apoptosis.

Preferably, at least 20% of cell death as a result of treatment by the methods described here is apoptotic. More preferably, at least 40%, 60%, 80% or more, most preferably greater than 95% of cell which die are apoptotic when treated for example with electric field and ultrasound.

The field strengths, time of application, and mode of application of the electric field and/or the ultrasound delivered by the apparatus may be manipulated or adjusted to achieve the desired proportion of cells which undergo death by apoptosis. Apoptosis may be assayed as described below. Thus, a high intensity, short duration exponential electric pulse may be delivered by the apparatus, for example, for apoptosis.

The term "high intensity" should be taken to refer to electric fields of between about 0.5 kV/cm and 3kV/cm, preferably between, about 1 kV/cm and 2kV/cm, more preferably, about 1.3 kV/cm. "Short duration" electric fields in the context of the above passage refer to between about 100 ms to 700 ms, preferably between about 250 ms to 450 ms, more preferably about 250 ms or 450 ms. For example, we disclose the use of a single or multiple electric pulse at 1.33 kV/cm, applied for between about 250 ms to 450 ms, in an exponential fashion, to achieve cell disruption by apoptosis.

"Necrosis" (also referred to as "accidental" cell death) refers to the pathological process which occurs when cells are exposed to a serious physical or chemical insult. Necrosis occurs when cells are exposed to extreme variance from physiological conditions (e.g., hypothermia, hypoxia) which may result in damage to the plasma membrane. Under physiological conditions direct damage to the plasma membrane is evoked by agents like complement and lytic viruses. Necrosis begins with an impairment of the cell's ability to maintain homeostasis, leading to an influx of water and extracellular ions. Intracellular organelles, most notably the mitochondria, and the entire cell swell and rupture (cell lysis). Due to the ultimate breakdown of the plasma membrane, the cytoplasmic contents including lysosomal enzymes are released into the extracellular fluid. Therefore, *in vivo,* necrotic cell death is often associated with extensive tissue damage resulting in an intense inflammatory response.

"Apoptosis" ("normal" or "programmed" cell death) refers to the physiological process by which unwanted or useless cells are eliminated during development and other normal biological processes. Apoptosis is a mode of cell death that occurs under normal physiological conditions and the cell is an active participant in its own demise ("cellular suicide"). It is most often found during normal cell turnover and tissue homeostasis, embryogenesis, induction and maintenance of immune tolerance, development of the nervous system and endocrine dependent tissue atrophy. Cells undergoing apoptosis show characteristic morphological and biochemical features. These features include chromatin aggregation, nuclear and cytoplasmic condensation, partition of cytoplasm and nucleus into membrane bound vesicles (apoptotic bodies) which contain ribosomes, morphologically intact mitochondria and nuclear material. *In vivo*, these apoptotic bodies are rapidly recognised and phagocytized by either macrophages or adjacent epithelial cells. Due to this efficient mechanism for the removal of apoptotic cells in vivo no inflammatory response is elicited. *In vitro,* the apoptotic bodies as well as the remaining cell fragments ultimately swell and finally lyse. This terminal phase of in vitro cell death has been termed "secondary necrosis".

Table 1 summarises the various observable differences between necrosis and apoptosis. Any of these differences, alone or in combination, may be assayed in order to determine whether cell death is occurring by apoptosis or by necrosis.

**Table 1 : Differential features and significance of necrosis and apoptosis.**

| | **Necrosis** | **Apoptosis** |
|---|---|---|
| **Morphological features** | Loss of membrane integrity Begins with swelling of cytoplasm and mitochondria Ends with total cell lysis No vesicle formation, complete lysis Disintegration (swelling) of organelles | Membrane blebbing, but no loss of integrity Aggregation of chromatin at the nuclear membrane Begins with shrinking of cytoplasm and condensation of nucleus Ends with fragmentation of cell into smaller bodies Formation of membrane bound vesicles (apoptotic bodies) Mitochondria become leaky due to pore formation involving proteins of the bcl-2 family. |
| **Biochemical features** | Loss of regulation of ion homeostasis No energy requirement (passive process, also occurs at 4°C) Random digestion of DNA (smear of DNA after agarose gel electrophoresis) Postlytic DNA fragmentation (= late event of death) | Tightly regulated process involving activation and enzymatic steps Energy (ATP)-dependent (active process, does not occur at 4°C) Non-random mono- and oligonucleosomal length fragmentation of DNA (Ladder pattern after agarose gel electrophoresis) Prelytic DNA fragmentation Release of various factors (cytochrome C, AIF) into cytoplasm by mitochondria Activation of caspase cascade Alterations in membrane asymmetry (i.e., translocation of phosphatidyl-serine from the cytoplasmic to the extracellular side of the membrane) |
| **Physiological significance** | Affects groups of contiguous cells Evoked by non-physiological disturbances (complement attack, lytic viruses, hypothermia, hypoxia, ischemica, metabolic poisons) Phagocytosis by macrophages Significant inflammatory response | Affects individual cells Induced by physiological stimuli (lack of growth factors, changes in hormonal environment) Phagocytosis by adjacent cells or macrophages No inflammatory response |

Reference is made to the following documents, which describe apoptosis in detail, as well as various assays for measuring cell death by apoptosis: Schwartzman, R. A. and Cidlowski, J. A. (1993). Endocrine Rev. 14, 133; Vermes, I. and Haanan, C. (1994). Adv. Clin. Chem. 31, 177; Berke, G. (1991). Immunol. Today 12, 396; Krähenbühl, O. and Tschopp, J. (1991). Immuno/. Today 12, 399; Van Furth, R. and Van Zwet, T. L. (1988). J. Immunol; Methods 108, 45. Cohen, J. J. (1993) Apoptosis. Immunol. Today14, 126; Savill, J. S. et al. (1989). J. Clin. Invest. 83, 865; Wyllie, A. H. (1980). Nature 284, 555; Leist, M. et al. (1994) Biochemica No. 3, 18-20; Fraser, A. and Evan, G. (1996) Cell 85, 781-784; Duke, R. C. (1983). Proc. Natl. Acad. Sci. USA 80,6361; Duke, R. C. & Cohen, J. J. (1986). Lymphokine Res. 5, 289; Trauth, B. C. et al. (1994) Eur. J. Cell. Biol. 63, 32,Suppl 40; Matzinger, P. (1991). J. Immunol; Methods 145, 185; Kaeck, M. R. (1993); Anal. Biochem. 208, 393; Prigent, P. et al. (1993). J. Immunol; Methods 160, 139; Huang, P. & Plunkett, W. (1992); Anal. Biochem. 207, 163; Bortner, C. D. et al. (1995) Trends Cell Biol. 5, 21; Gold, R. et al. (1994); Lab. Invest. 71, 219.

Apoptosis and cell mediated cytotoxicity are characterised by cleavage of the genomic DNA into discrete fragments prior to membrane disintegration. Accordingly, apoptosis may be assayed by measuring DNA fragmentation, for example, by observing the presence of DNA ladders. DNA fragments may be assayed, for example, as "ladders" (with the 180 bp multiples as "rungs" of the ladder) derived from populations of cells, or by quantification of histone complexed DNA fragments via, for example, ELISA. Such an assay relies on an one-step sandwich immunoassay to detect nucleosomes. The procedure involves pelleting cells by centrifugation and discarding the supernatant (which contains DNA from necrotic cells that leaked through the membrane during incubation). Cells are resuspended and incubated in lysis buffer. After lysis, intact nuclei are pelleted by centrifugation. An aliquot of the supernatant is transferred to a streptavidin-coated well of a microtiter plate, and nucleosomes in the supernatant are bound with two monoclonal antibodies, anti-histone (biotin-labelled) and anti-DNA (peroxidase-conjugated). Antibody-nucleosome complexes are bound to the microtiter plate by the streptavidin. The immobilised antibody-histone complexes are washed three times to remove cell components that are not immuno-reactive, and the sample is incubated with peroxidase substrate (ABTS ®). The amount of coloured product (and thus, of immobilized antibody- histone complexes) is then determined spectrophotometrically.

Several proteases are involved in the early stages of apoptosis. Apoptosis may therefore also be assayed by detecting the presence of, in addition to, or instead of, assaying the activity of, apoptosis-induced proteases such as caspases, e.g., caspase 3. Caspase activation can be analyzed in different ways, for example, by an *in vitro* enzyme assay of, for example, cellular lysates by capturing of the caspase and measuring proteolytic cleavage of a suitable substrate. Furthermore, caspases may be assayed by detection of cleavage of an *in vivo* caspase substrate such as PARP (Poly-ADP-Ribose-Polymer-ase). Cleaved fragments of PARP may be detected with a suitable antibody such as an anti PARP antibody. Protease assays and DNA fragmentation assays are especially suitable for assaying apoptosis in cell populations.

Methods for studying apoptosis in individual cells are also available, such as ISNT and TUNEL enzymatic labeling assays. As noted above, extensive DNA degradation is a characteristic event which often occurs in the early stages of apoptosis. Cleavage of the DNA yields double-stranded, low molecular weight DNA fragments (mono- and oligonucleosomes) as well as single strand breaks ("nicks") in high molecular weight-DNA. In TUNEL, such DNA strand breaks are detected by enzymatic labeling of the free 3'-OH termini with suitable modified nucleotides (such as X-dUTP, X = biotin, DIG or fluorescein). Suitable labeling enzymes include DNA polymerase (nick translation) in ISNT (*"in situ* nick translation") and terminal deoxynucleotidyl transferase (end labeling) in TUNEL ("TdT-mediated X-DUTP nick end labeling"; Huang, P. & Plunkett, W., 1992, Anal. Biochem. 207, 163; Bortner, C. D. et al., 1995, Trends Cell Biol. 5, 21).

Apoptosis may also be assayed by measuring membrane alterations, including: loss of terminal sialic acid residues from the side chains of cell surface glycoproteins, exposing new sugar residues; emergence of surface glycoproteins that may serve as receptors for macrophage-secreted adhesive molecules such as thrombospondin; and loss of asymmetry in cell membrane phospholipids, altering both the hydrophobicity and charge of the membrane surface. In particular, the human anticoagulant annexin V is a 35-36 kilodalton, Ca2+-dependent phospholipid-binding protein that has a high affinity for phosphatidylserine (PS). In normal viable cells, PS is located on the cytoplasmic surface of the cell membrane. However, in apoptotic cells, PS is translocated from the inner to the outer leaflet of the plasma membrane, thus exposing PS to the external cellular environment. Annexin V may therefore be used to detect phos-phatidylserine asymmetrically exposed on the surface of apoptotic cells (Homburg, C. H. E. et al. 1995, Blood 85, 532; Verhoven, B. et al., 1995, J. Exp. Med. 182, 1597). Furthermore, DNA stains such as DAPI, ethidium bromide and propidium iodide, etc may be used for differential staining to distinguish viable and non-viable cells. Profiles of DNA content may also be used; thus, permeabilized apoptotic cells leak low molecular weight DNA, and detection of "sub-G 1 peaks", or "A 0 " cells (cells with lower DNA staining than that of G 1 cells) may be detected by, for example, flow cytometry. Morphological changes characteristic of apoptosis may also be detected in this manner.

Detection of apoptosis-related proteins such as ced-3, ced-4, ced-9 (Ellis, H. M. and Horvitz, H. R., 1986, Cell 44, 817-829; Yuan, J. Y. and Horvitz, H. R., 1990, Dev. Biol. 138, 33-41; Hentgartner, M. O., Ellis, R. E. and Horvitz, H. R., 1992, Nature 356, 494-499.), Fas(CD95/Apo-1; Enari et al., 1996, Nature 380, 723-726), Bcl-2 (Baffy, G. et al., 1993, J. Biol. Chem. 268, 6511-6519; Miyashita, T. and Reed, J. C., 1993, Blood 81, 151-157; Oltvai, Z. N., Milliman, C. L. and Korsmeyer, S. J., 1993, Cell 74, 609-619), p53 (Yonish-Rouach, E. et al., 1991, Nature 352, 345-347), etc by the use of antibodies may also be used to assay apoptosis.

### PHARMACEUTICAL COMPOSITIONS

Where an embodiment of the apparatus is employed which is capable of delivering a cell-death facilitating agent in addition to ultrasound and electric field, the composition comprising the cell-death facilitating agent or agents may be administered alone. However, and preferably, the cell-death facilitating agent is formulated and delivered by the apparatus as a pharmaceutical formulation. Such a pharmaceutical composition is preferably contained in a reservoir in the apparatus, and delivered by suitable means, for example, by being carried in hollow needles as described above.

The pharmaceutical composition may include the cell-death facilitating agent, a structurally related compound, or an acidic salt thereof. The pharmaceutical formulations disclosed comprise an effective amount of cell-death facilitating agent together with one or more pharmaceutically-acceptable carriers. The effective amount will vary depending upon the particular conditions of treatment, cell type, as well as other factors including the age and weight of the patient, the general health of the patient, the severity of the symptoms, and whether the cell-death facilitating agent is being administered alone or in combination with other therapies.

Suitable pharmaceutically acceptable carriers are well known in the art and vary with the desired form and mode of administration of the pharmaceutical formulation. For example, they can include diluents or excipients such as fillers, binders, wetting agents, disintegrators, surface-active agents, lubricants and the like. Typically, the carrier is a solid, a liquid or a vaporizable carrier, or a combination thereof. Each carrier should be "acceptable" in the sense of being compatible with the other ingredients in the formulation and not injurious to the patient. The carrier should be biologically acceptable without eliciting an adverse reaction (e.g. immune response) when administered to the host.

The pharmaceutical compositions of use may comprise a soak, an ointment or water-in-oil emulsion, a cream, a lotion, a paste, a gel, a stick, a spray, an aerosol, a bath oil, a solution and the like.

In general, the concentration of the cell-death facilitating agent in the formulation is in an amount of about 0.5 to 50% by weight of the composition, preferably about 1 to 30%, more preferably about 2-20%, and most preferably about 5-10%. The concentration used can be in the upper portion of the range initially, as treatment continues, the concentration can be lowered or the application of the formulation may be less frequent.

For some applications, it is preferable to administer a long acting form of cell-death facilitating agent using formulations known in the art, such as polymers.

### ASSAYS

The observation that cells exposed to electric fields and ultrasound exhibit cell death through apoptosis may be used as the basis of assays to identify useful molecules and targets for therapy.

One such assay seeks to identify molecules such as compounds which are capable of modulating a process involved in apoptosis of a cell. For example, assays which identify molecules which modulate (i.e., promote or inhibit) caspases or other apoptosis protease activity are envisaged. The assays in general involve contacting a cell with a candidate molecule, i.e., a molecule or compound which is suspected of having apoptosis modulatory activity. Such candidate molecules may be provided in the form of libraries such as combinatorial libraries as known in the art.

The cell is then treated by exposure to a sensitiser (e.g., an electric field) followed by a disrupter (e.g., ultrasound) delivered by the apparatus as described above, at levels which are known, or have been determined to be, capable of inducing apoptosis in cells of the particular type or characteristics, etc. The progress or degree of apoptosis is observed to identify molecules which are capable of enhancing, promoting, inhibiting or stopping apoptosis of the cells. Molecules identified by such an assay are useful as drugs to enhance or inhibit apoptotic cell death, and may be used as therapies of diseases in which apoptotic cell death is exhibited.

Another assay is capable of identifying genes which are involved in regulating apoptosis, or genes which are involved in apoptotic processes. Such an assay relies essentially on modulating the function of a gene or gene product which is suspected of being involved in, or involved in regulating, an apoptosis process. By gene product, we mean an RNA transcribed from the gene, or a polypeptide product of the gene. For example, gene function may be disrupted by mutation, as known in the art, or by use of a known inhibitor of a gene product, for example, antisense RNA, or a chemical inhibitor. The cell is then exposed to a sensitiser and a disrupter (e.g., an electric field and ultrasound), delivered by the apparatus as described above, at levels which are known, or have been determined to be, capable of inducing apoptosis in cells of the particular type or characteristics, etc, and the presence, degree or rate of apoptosis observed. Similarly, gene function may be enhanced and apoptosis assayed. Candidate genes which are suspected of being involved in apoptosis may then be used as potential targets for drug discovery programs, to identify candidate modulators of apoptosis (for example, by use of the above assay).

The following examples demonstrate that a combination of electric field and ultrasound delivered to a cell causes its disruption or ablation.

### EXAMPLES

### Example 1. The Effect of Low Intensity Ultrasound on Cells Treated with Pulses of 3.625kV/cm

The target cell line employed in these studies is a mouse friend leukaemic lymphoblast cell line (clone 707, ECACC no. 91112126 from the European Collection of Animal Cell Cultures) and is maintained in DMEM supplemented with 10% (v/v) foetal bovine serum. Cultures are maintained in a humidified 5% CO₂ atmosphere at 37°C. Cells are harvested by centrifugation, washed once in phosphate buffered saline (PBS) and suspended at a concentration of 1.065 x 10⁷ cells/ml. 0.7ml aliquots of this suspension are dispensed into electroporation cuvettes (0.4cm electrode gap) together with 0.1ml of PBS. Cuvettes are retained on ice and electroporated by delivering two pulses of 3.625kV/cm at a capacitance of 1µF. Cells are washed twice in PBS by centrifugation, resuspended in PBS containing MgCl₂ (4mM) (PBS/Mg) and retained at room temperature for 30min. Cells are washed twice in PBS/Mg containing 10mM glucose, resuspended in the same buffer and retained at room temperature for 1 hour. A control population of cells is taken through the same procedure except that the electroporation step is omitted. Cell concentrations are adjusted to 1.4 x10⁷ cells/ml. 100µl aliquots of cells are dispensed into microwells from a 96-well plate and positioned on a 3MHz ultrasound head. Cells are exposed to ultrasound for 30secs. Viability is determined using trypan blue.

The effect of increasing ultrasound power density on cell viability of normal cells and electrosensitised cells is shown in Figure 19. The results demonstrate little or no effect on the normal control population of cells up to a power density of 1.5W/cm² whereas cell viability decreased to almost 0 at 1 W/cm² following treatment of the electro-sensitised population. It should be noted that cell viability is determined immediately after exposure to ultrasound. These results demonstrate that it is possible to sensitise the target cells to ultrasound conditions that have no effect on normal cells.

### Example 2. The Effect of Low Intensity Ultrasound on Cells Exposed to Pulses of 1.875 and 2.5 kV/cm

In order to determine whether or not the pulse electric field strength had any effect on susceptibility of the treated cells to low intensity ultrasound, 0.7ml aliquots of cells (0.8 x 10⁷ cells/ml in PBS/Mg) are dispensed into electroporation cuvettes (0.4cm electrode gap) together with 0.1ml of PBS. Cuvettes are retained at room temperature and electroporated as described for Example 1 except that one population is treated with two pulses at 1.875kV/cm and another is treated with two pulses at 2.5kV/cm. Cells are transferred to PBS/Mg/glucose and retained at room temperature for 15min. Samples are treated with ultrasound for 30sec. and allowed to stand at room temperature for 1 hour prior to determination of cell viability using trypan blue. A control population of cells is taken through the above treatment except that the electroporation event is omitted.

The effect of low intensity ultrasound on cells treated at both voltages is shown in Figure 20. In this case ultrasound had a limited effect on cell viability in the control population of cells up to 0.75W/cm². At or above 1W/cm² viability decreased dramatically in the control population. Ultrasound-mediated effects are observed in the population of cells treated with electric pulses of 1.875kV/cm and viability is decreased at the lower ultrasound densities (0.25 - 0.75W/cm²). In the cells treated at 2.5kV/cm² stronger effects are noted at all ultrasound power densities examined between 0.25 and 1 W/cm². The results confirmed that ultrasound sensitivity could be induced by exposure of cell populations to electric pulses. The results also demonstrate that susceptibility of cells to ultrasound increased with increasing electric field strength. The decrease in control cell viability following treatment with ultrasound in this experiment is more dramatic than that observed in Example 1 (Figure 19) and this may be due to the increased resting time between ultrasound treatment and determination of cell viability described in Example 2.

### Example 3. Sensitisation and Ultrasound Treatment of Cells Immobilised in Alginate Matrices

In order to determine whether or not this sensitisation phenomenon could be achieved in a mass of cells, thereby mimicking a tumour mass, it was decided to embed the cells in an alginate matrix, expose the mass to electric pulses and subsequently expose it to ultrasound. Viability could then be determined using a modification of the MTT assay described previously (Rollan et al., Bioprocess Eng. 15, 47, 1996). To the above end 707 cells are harvested and suspended in 1% (w/v) sodium alginate (Keltone LV, Lot no. 35245A, Kelco, UK) at a concentration of 1.16 x 10⁷ cells/ml. This suspension is added drop-wise to a calcium chloride solution (1.5% [w/v]) and beads (average vol. per bead = 10µl) retained in CaCl₂ for 15min. Beads are subsequently rinsed in PBS and dispensed into electroporation cuvettes (30 beads /cuvette) together with 0.5ml PBS. Two electric pulses of 2.5kV/cm at a capacitance of 1 µF are delivered to each cuvette and cells are immediately transferred to culture medium. Aliquots of 5 beads are dispensed into the wells of a 96-well plate and exposed to ultrasound at 0.75 and 1.5W/cm² at 3MHz for 40 seconds. Beads are then placed in an incubator at 37°C for 165 minutes. Medium is subsequently removed and the beads are washed once in PBS. 1ml aliquots of MTT (1mg/ml in PBS) are added to each sample of beads and these are retained at 37°C for 1 hour. The MTT is then removed from the beads and 0.5ml of NaOH (1M) is added to each sample. Viability of cells in the beads is determined by measuring the absorbance of the resulting solution at 520nm using spectophotometry. Control samples consisted of immobilised cells taken through the procedure with the exception of exposure to either electric pulses or ultrasound.

The results from these experiments are shown in Figure 21 and they demonstrate that ultrasound exposure at either 0.75 or 1.5W/cm² had a very limited effect on control cells which had not been exposed to the electric pulses. Exposure of cells to electric pulses in the absence of ultrasound treatment resulted in a 50% decrease in viability. However, treatment of electro-sensitised cells with ultrasound at both power densities had dramatic effects on cell viability where treatment of samples at 1.5W/cm² resulted in an 84% reduction in cell viability. It is important to note that the same power density had little or no effect on cell viability in the control sample. The results presented here demonstrate that a mass of cells may be sensitised to ultrasound using electric pulses and suggests that this may also be the case in a tissue mass *in vivo.*

The above results demonstrate that subjecting cell populations to electric fields in culture render those cells sensitive to low intensity ultrasound.

The following examples demonstrate that when tissue masses are treated with electric fields *in vivo,* those tissues are sensitive to low intensity ultrasound. These examples make use of a mouse tumour model known as RIF-1 (Twentyman et al., 1980, J. Natl. Cancer Inst., 64 595-604). In Example 4, tumour cells are treated *in vitro* and these treated populations are then employed to inoculate animals. The development of tumours is monitored. In Example 5, animals are inoculated with the cells and the tumours which develop are treated with electric fields *in vivo* and subsequently with ultrasound.

### Example 4. Tumour Development Following Electrosensitisation and Ultrasound Treatment of Tumour Cells in vitro

In this experiment RIF-1 cells are treated with electric fields, ultrasound or a combination of both, and the ability of the treated populations to induce tumour growth is assessed.

The target cells are grown in RPMI 1640 medium supplemented with 10 (v/v) foetal bovine serum and 1% penicillin/streptomycin stock (5000u/ml and 5000µg/ml, respectively) and in a 5% CO₂ humidified atmosphere. Cells are cultured to confluence and then harvested following treatment with trypsin-EDTA (0.005% and 0.002% [w/v], respectively). Cell concentrations are adjusted to 1 x 10⁶ cells/ml in phosphate buffered saline and 0.8ml aliquots are treated with (i) electric fields alone [1kV/cm, double pulse at 1µF], (ii) ultrasound [1.25W/cm² at 3MHz for 30 sec.] and (iii) electric fields followed immediately by treatment with ultrasound. Control populations consist of cells at the same concentration without treatment. These cell populations are then used to inoculate 8-week old male C3H mice by intradermal injection of 0.1ml into the rear dorsum of each animal. Tumour volume is calculated from the geometric mean of the diameter measured in 3 dimensions using the formula 4/3 π r³.

### Example 4 Results

The results are shown in Figure 22 and they demonstrate that whilst ultrasound has little or no effect on the ability of the cells to induce tumour growth, electrosensitisation does have a significant effect in this regard. The latter effect has been shown in for example Mir et al., 1991, Eur J. Cancer, 27, 68-72.

However, most notably, tumours derived from the cells, which receive the combined electric field and ultrasound, fail to give rise to tumours until day 17. The results demonstrate that the combined treatment has the most dramatic effect on induction of tumour formation and they further suggest a degree of synergy between treatments.

### Example 5. Effect of Continuous Wave and Pulse Wave Ultrasound on Electrosensitised Tumour Cells in vivo

In this series of experiments tumours are induced in animals and these are employed as targets to determine the effects of *in vivo* treatments using electric fields, ultrasound (both continuous wave and pulsed) and combined treatments with electric fields followed by ultrasound.

To this end animals are inoculated with RIF-1 tumour cells as described above. When tumours reach an average volume of 50mm³ they are either untreated (control), treated with electric fields (set voltage =1.66kV/cm and delivered voltage = 1.33kV/cm using a BTX 630 system together with Tweezertrodes, 7mm), ultrasound on continuous wave emission at 3MHz and at 0.7W/cm², ultrasound on pulsed wave emission at 3MHz at 1.8W/cm² (duty cycle of 35%) and combinations of the electric field followed immediately by each form of ultrasound. Tumour growth is monitored by measuring tumour volume and this is determined as described above.

### Example 5 Results

The results are shown in Figure 23 and they demonstrate that pulsed wave ultrasound treatment alone has no effect on tumour growth. Both electric field treatment alone and continuous wave ultrasound alone appear to have a slight effect on tumour growth. Notably, the combined treatments of electric field and each type of ultrasound show the greatest inhibition of tumour development.

In tumours which are treated with electric fields combined with ultrasound, those treated with pulsed wave ultrasound exhibit the greatest response, although the negative effects on growth following combined treatment with continuous wave ultrasound are also significant. It should be noted, however, that in terms of total energy delivered to the cells, pulsed wave ultrasound appears to be slightly more efficient than continuous wave (78 J/cm² for pulsed wave ultrasound versus 84 J/cm² for continuous wave ultrasound).

These results demonstrate that tumours treated with electric pulses *in vivo* are rendered sensitive to relatively low intensity ultrasound.

### Example 6. Sensitisation of Tumour Cells to Ultrasound in vivo Using Square Wave Electric Pulses

This Example demonstrates the ability of high intensity and short duration square wave electric pulses to sensitise tumour cells to ultrasound *in vivo.*

In the above series of experiments is shown that exposing tumours *in vivo* renders them sensitive to relatively low-intensity ultrasound. In those experiments the manner in which the electric field is delivered to the tissues includes both high-intensity, short duration exponential electric pulses and low intensity direct current for a prolonged period of time.

In order to determine whether or not conditions using short -duration and high intensity square-wave pulses can be employed to facilitate hyper-sensitisation of tumour tissues to ultrasound, tumours are treated with conditions using short -duration and high intensity square-wave pulses and sensitivity to ultrasound determined. To this end a series of tumours are established in animals (n=4 per group) and three groups of animals are treated with 8 square wave pulses consisting of 100 µsecond duration, an electric field strength of 1.25kV/cm², delivered at a frequency of 1Hz. One of these groups is then subjected to treatment immediately with pulsed wave ultrasound (35% continuous wave) for 2 min. at 3.57W/cm² and at 1MHz. Another group receives ultrasound 24 hours after delivery of the electric field and a third group receives no ultrasound. In addition, a control untreated group of animals is employed in the experiment as is a group treated with ultrasound alone. Tumour growth is monitored as described previously.

### Example 6 Results

The results obtained from these experiments are shown in Figure 24 and they demonstrate that treatment with the electric field alone has a significant effect on tumour growth, as expected (Mir et al.,., Eur J. Cancer, 22, 1991, 68-72). When tumours are treated with the electric fields and immediately thereafter with ultrasound, a significant reduction in tumour growth is observed, although this is not as dramatic as the effect observed using exponential wave or DC treatment. When tumours are treated with ultrasound 24 hours after electric field treatment no significant effect on tumour growth retardation is observed. These results suggest that the electric field conditions short - duration and high intensity square-wave pulses are not as efficient as either exponential wave or DC at sensitising tumours to ultrasound *in vivo*.

It is however, worth noting that in our studies with high intensity exponential wave treatments the time constants observed during delivery of pulses is in the region of 300-400msec., whereas the square wave pulses employed here are in the region of 100µsec. In accordance with this, experiments similar to the above, but in which the square wave pulse duration is increased from the µsecond range to the millisecond range show more dramatic responses in reduction of tumour growth.

### Example 7. Effect of Higher Intensity Continuous Wave and Pulse Wave Ultrasound on Electrosensitised Tumour Cells in vivo

Animals are inoculated with RIF-1 tumour cells, tumours treated, and tumour growth monitored as described above in Example 5. Treatment comprises electric fields (1.33kV/cm), ultrasound on continuous wave emission at 3MHz and at 1.25W/cm² for 2 minutes, ultrasound on pulsed wave emission at 3MHz at 2.5W/cm² for 2 minutes (35% continuous wave) and combinations of the electric field followed immediately by each form of ultrasound. Untreated cells are used as control, as above.

### Example 7 Results

The results are shown in Figure 25 and they demonstrate that both pulsed wave ultrasound treatment and continuous wave treatment of electrosensitised cells are effective in slowing tumour growth. As before, pulsed wave treatment appears to be more effective than continuous wave treatment, even though the total energy delivered to the cells is 105 J/cm² for pulsed wave ultrasound versus 150 J/cm² for continuous wave ultrasound.

These results demonstrate that tumours treated with electric pulses *in vivo* are rendered sensitive to higher intensities of ultrasound.

### Example 8. Treatment of Electrosensitised Tumours with Ultrasound At Various Times After the Electrosensitisation Event

In the previous Examples ultrasound treatment immediately follows electrosensitisation. In order to examine the length of time tumours remain sensitive to ultrasound after the electrosensitisation event, we decided to electrosensitise tumours *in vivo* and treat those tumours with ultrasound at various times after electrosensitisation. To this end tumours are inoculated into recipient mice as described above. Those tumours are electrosensitised by exposure to double pulses at 1.33kV/cm. Tumours are then exposed to ultrasound (3.57W/cm², 1MHz using pulsed wave at 35% continuous wave for 2 min.) at 0, 0.5, 1, 2, 6 and 18 hours after electrosensitisation. Control animals remain untreated or are exposed to either electric field or ultrasound treatment alone. Tumour volume is monitored following treatment as described above.

### Example 8 Results

The results obtained from this experiment are shown in Figure 26 and they demonstrate that significant effects are detected when ultrasound is delivered at all times post electrosensitisation. These results demonstrate that cells remain electrosensitised to ultrasound for a considerable period of time following electrosensitisation. The results demonstrate that ultrasound does not necessarily have to be delivered immediately after electrosensitisation for cell ablation to take place.

### Example 9. Induction of Apoptosis in Cells Treated with Electric Field and Ultrasound

In order to examine the molecular mechanism by which combined exposure of cells to electric fields and ultrasound induces cell death, cells are treated with a single pulse at various voltages and the effects of ultrasound on those cells are examined.

In addition to examining cell numbers remaining following treatment we determine whether or not the remaining population of cells are apoptotic or necrotic. To this end 707 cells are harvested and suspended in PBS at a concentration of 1.53x10⁶ cells/ml. 0.8ml aliquots are dispensed into electroporation cuvettes (0.4cm electrode gap) and cells are treated with single electric pulses at a capacitance of 1µF. Cells are harvested from cuvettes and each 0.8ml aliquot is washed by centrifugation and resuspended in 2ml of tissue culture medium containing foetal bovine serum. Each 2ml aliquot is dispensed into a 2ml well of a 24-well tissue culture plate. Control populations of cells are not treated with electric pulses but are dispensed into 2ml wells. All samples are treated with ultrasound for 30 seconds at a power density of 1.25W/cm² and using a 3MHz ultrasound head (single pulse). Cells are then incubated at 37°C for 21 hours in a humidified 5% CO₂ atmosphere. Following incubation, cells are harvested and the proportion of cells which are either apoptotic or necrotic is determined by staining with an Annexin-V-FLOUS staining kit (Roche, UK). Following staining cells are suspended in HEPES buffer and analysed using flow cytometry.

### Example 9 Results

The results obtained are shown in Figure 27. They demonstrate that ultrasound treatment alone results in an approximate 20% decrease in cell numbers. As the voltage is increased, combined treatment leads to a very significant decrease in cell numbers and this finally reaches a steady state above voltages of 750V.

In addition to examining cell numbers in remaining populations, the proportion of both apoptotic and necrotic cells in those surviving populations is examined. The results are also shown in Figure 27 and they demonstrate that a significantly greater proportion of surviving cells remaining following treatment are apoptotic. These results suggest that treatment of electrosensitised cells with ultrasound facilitates the onset of apoptosis rather than necrosis.

### Example 10. Induction of Apoptosis in Tumours Following Treatment with Combined Electric Fields and Ultrasound in Vivo

As shown in previous Examples, treatment of target cell populations with electric fields and ultrasound leads to the induction of apoptosis *in vitro.* The objective of this Example is to demonstrate that combined treatment with electric fields followed by treatment with ultrasound *in vivo* leads to induction of apoptosis.

To the above end RIF-1 tumours are induced in C3H mice and these are employed as target tumours for combined treatments with electric pulses followed by ultrasound. Control animals receive no treatment. Conditions used in electrosensitisation involve treatment with a double pulse regime consisting of 1.33kV/cm and ultrasound treatment involve the use of pulsed wave ultrasound (35% continuous wave) at 1MHz, 3.57W/cm² for 2 min. Following treatment, tumours from control, untreated animals and those receiving treatment are harvested at 0, 6, 12,18 and 24 hours post treatment. After harvesting tumours are fixed in 4% (w/v) paraformaldehyde overnight. Paraffin wax sections are then prepared for each sample and these are stained using the *In situ* cell death detection kit, TMR red (Roche, UK) according to the manufacturer's instructions. This staining method is based on terminal deoxynucleotidyl transferase nick end labelling (TUNEL) and apoptosis is indicated by fluorescent staining as observed using fluorescence microscopy.

### Example 10 Results

When sections are stained for apoptosis the results obtained following examination with fluorescence microscopy are shown in Figure 28. In control samples, the only sample that exhibits slight positive staining is the 24 hours sample and this may result from the inclusion of normal cells in the sections. All other control samples fail to yield a signal for apoptosis. In the treated samples, staining for apoptosis becomes strongly evident at 12 hours following treatment and clear signals are also evident at 18 and 24 hours (Figure 28). The results clearly demonstrate that combined treatment with electric fields and ultrasound results in the onset of apoptosis.

### Example 11. Effect of Treating Tumours with Ultrasound Prior to Exposure to Electric Field in Vivo

In this series of experiments tumours are established in mice as described above for Example 5. However in this Example, the tumours are treated with ultrasound prior to treatment with electric fields.

In this study animals are inoculated with RIF-1 tumours as described above. Tumours are then treated with ultrasound (2 min.) at 1MHz using a power density of 1.25W/cm² at continuous wave and 3.57W/cm² at pulsed wave (35% continuous wave) delivery mode. Tumours are then treated immediately with electric fields (double pulse using set voltage = 1.66kV/cm using a BTX 630 system together with Tweezertrodes, 7mm). Tumour growth is monitored by measuring tumour volume and this is determined as above.

### Example 11 Results

The results are shown in Figure 29 and they demonstrate that treatment of tumours with ultrasound prior to electric fields also has an inhibitory effect on tumour growth. In addition, the inhibitory effect obtained using pulsed wave ultrasound is again greater than that observed using continuos wave ultrasound.

Surprisingly, the results demonstrate that the advantage associated with the use of combined treatment with electric fields and ultrasound in terms of tumour treatment is realised whether ultrasound is delivered prior to or following treatment with electric fields.

### Example 12. Effect of Low Voltage Direct Current Electricity Treatment

Relatively intense electric fields are employed in the above examples. However, anti-tumour effects have also been demonstrated following treatment of tumours with low voltage/direct current (DC) alone (Nordenstrom, Am. J. Clin. Oncol. 1989, 12, 530-536; Wojcicki et al., Med Sci. Monit. 2000, 6, 498-502). We decided to investigate whether such low voltage/DC can render tumours or other cells hyper-sensitive to relatively low intensity ultrasound.

RIF-1 tumours are established in C3H recipient mice as described above. Needles are then horizontally inserted on each side of the tumours and electrodes attached to the needles. A constant current of 5mA is established across the needles for a period of 15 min. and are treated immediately with ultrasound at 3.75W/cm² for a period of 3 minutes. During the treatment the electric field strength ranges from 10 to 20V/cm, with the field strength increasing as treatment progresses. Control animals are treated with electric current alone. Tumour volume is then monitored as described above.

### Example 12 Results

The results are shown in Figure 30 and they demonstrate that in the animal that is treated with electric current alone, the tumour volume decreases significantly over the time period examined. This reaches a minimum at 13 days. This also occurs in the animal that is treated with both electric current and ultrasound. However, the rate at which tumour volume decreases is significantly higher. In this case tumour volume reaches a minimum within 4-6 days.

It should be noted that the starting tumour size in this experiment is much greater than that in other studies described previously and in this context, the decrease in tumour size observed here is very dramatic. These results demonstrate that, although electrosensitisation of tumours to relatively low intensity ultrasound is achieved using pulses of high electric field strength, this phenomenon also occurs using strategies employing low electric field strengths with direct current. This observation broadens the degree of utility of our technology, particularly in cases where large areas of tissue may need to be sensitised.

### Example 13. Treatment of Tumours with Direct Current Together with Ultrasound At 5W/Cm² and 1Mhz

The objective of the experiments in this Example is to examine the effects of ultrasound at increased intensity on direct current-electrosensitised tumours. To this end, RIF-1 tumours are established in recipient C3H mice as described above and treated with (i) direct current alone at 5mA for 5 min, (ii) pulsed ultrasound alone at 5W/cm² for 2 min. at 35% continuous wave, and (iii) direct current plus pulsed ultrasound using the conditions listed above. Tumour volume is measured as described previously. In addition the growth of control, untreated tumours is also monitored.

### Example 13 Results

The results are shown in Figure 31 and they demonstrate that treatment with direct electric current leads to a decrease in tumour volume, although this begins to increase again at days 4 to 5. Treatment with ultrasound has a slight effect on tumour growth although at no stage is a reduction in tumour volume detected. In the group of animals receiving the combined treatment with direct current and ultrasound, complete regression is observed and this continues to be the case within the time period examined. The results again demonstrate that combined treatment of tumours with direct electric current and ultrasound leads to dramatic tumour regression.

### Example 14. Treatment of Tumours with Direct Current Together with Ultrasound (Six Animals)

The intention in this series of experiments described in this Example is to confirm the result obtained in the previous Example (DC treatment) and to examine the fate of animals receiving combined treatments over an extended period of time. In this case 6 animals are used in each group and both the DC and ultrasound treatments are similar to those described in Example 13. After treatment, tumour growth is measured in each group as described previously.

### Example 14 Results

The results obtained are shown in Figure 32 and they demonstrate again, that a significant reduction in tumour growth is observed when animals are treated with the electric field alone. In this case the tumour mass is eradicated following treatment, although growth begins to appear in all animals after day 4.

In the group receiving combined ultrasound and electric field treatment the tumour masses are again eradicated. When this group of animals is monitored for a prolonged period of time three animals begin to exhibit growth at around day 14 and these animals are eventually sacrificed at day 23. On day 27 two further animals begin to exhibit measurable growth and these are terminated on day 35. Subsequently, the one remaining animal fully recovers and remains disease free throughout the period of time indicated in Figure 32. Although five out of six animals exhibit tumour growth following combined treatment, the survival of one disease-free animal demonstrates that this approach can be used in the treatment of aggressive disease.

### Example 15. Ultrasound Treatment of Tumours 22 Hours after Direct Current Treatment

In previous experiments it is demonstrated that treating tumours with ultrasound at extended times after delivery of the electric pulses yields an increased effect in terms of retarding tumour growth. In order to determine whether or not this applies to treatment with DC, animals with tumours are treated with DC (5mA for 5 min.) and allowed to rest for a period of 22 hours. At this stage animals receiving the combined treatments are exposed to ultrasound (5W/cm² @ 1MHz for 2min. and pulsed at 35% continuous wave). In these experiments four animals are employed per group and tumour growth is monitored following treatment as described above.

### Example 15 Results

The results from these experiments are shown in Figure 33 and they again confirm a significant effect of DC treatment on tumour growth. However, as before, tumours begin to re-appear in these animals after day 4. In the group receiving the DC treatment followed by ultrasound treatment at 22h, the tumour masses are again completely eradicated. When these animals are monitored, growth in one animal appears after day 14 and this animal is terminated at day 20. Growth appears in another animal from this group after day 3 0 and this is terminated on day 37. In this group of animals receiving the combined treatment, two animals out of 4 remain disease-free throughout the duration of the experiment.

These results demonstrate the positive effects of DC treatment combined with ultrasound in terms of tumour eradication. In addition, the results also demonstrate that tumour cells remaining after the DC treatment remain sensitive to ultrasound *in vivo* for at least 22 hours.

### FURTHER ASPECTS

Further aspects of the invention are now set out in the following section and numbered paragraphs; it is to be understood that the invention encompasses these aspects:

We provide an apparatus for ablating a cell, preferably a nucleated cell, or a tissue comprising such a cell, the apparatus comprising: (a) electric field generating means; an (b) ultrasound generating means.

One or both of the electric field generating and the ultrasound generating means may comprise a head portion which is capable of being positioned such that it is in proximity to the cell or tissue to be ablated. The electric field generating means may be capable of generating electric field energy to electrosensitise a cell, preferably a nucleated cell, such that it is rendered more susceptible to disruption by ultrasound than an unsensitised cell. Preferably, the electric field generating comprises an electric field generator capable of generating an electric field from about 1 Volt/cm to about 10 kVolts/cm under *in vivo* conditions. Furthermore, the ultrasound generating means is preferably capable of generating ultrasound energy to selectively disrupt a cell in an organism, in which the cell has previously been electrosensitised by exposure to electric field energy. Furthermore, the ultrasound generating means is preferably capable of generating ultrasound at a power level of from about 0.05W/cm² to about 1.00W/cm².

We further describe a device or an apparatus suitable for ablating a cell, for example within an organism.

In general, the device comprises a sensitisation means and a disruption means, in which the sensitisation means is capable of sensitising a cell, preferably a nucleated cell,. Such a sensitised cell is rendered more susceptible to disruption by an energy source, compared to a cell, preferably a nucleated cell, which has not been so sensitised. The disruption means is capable of generating energy at a frequency and/or energy sufficient to disrupt sensitised cells, preferably sensitised nucleated cells, preferably at a frequency and/or energy which is at the same time insufficient to disrupt unsensitised cells. Accordingly, the device comprises an electric field generating means and an ultrasound generating means.

In one embodiment, the sensitisation means comprises an electric field generating means, while the disruption means comprises an ultrasound generating means. In this embodiment, the electric field generating means preferably comprises an electrosensitisation means which is capable of sensitising a cell, preferably a nucleated cell, to render it susceptible to disruption by an energy source. Preferably, the electrosensitisation means is capable of generating electric field energy to electrosensitise a cell, preferably a nucleated cell, such that it is rendered more susceptible to disruption by ultrasound than an unsensitised cell. Preferably, the electrosensitisation means generates electric field energy to electrosensitise a cell, preferably a nucleated cell, to ultrasound energy in an organism, in which the electrosensitised cell is rendered susceptible to ultrasound disruption at a frequency and energy sufficient to cause disruption of the electrosensitised cell but insufficient to cause disruption of unsensitised cells.

The ultrasound generating means is preferably capable of generating ultrasound energy to selectively disrupt a cell in an organism, in which the cell has previously been electrosensitised by exposure to electric field energy. The ultrasound generator more preferably generates ultrasound energy to selectively disrupt a cell in an organism, in which the cell has previously been electrosensitised by exposure to electric field energy, and the ultrasound is delivered at a frequency and energy sufficient to cause disruption of the electrosensitised cell but insufficient to cause disruption of unsensitised cells.

The electric field generating means preferably is capable of generating an electric field, preferably a pulse, from about 1 Volt/cm to about 10 kVolts/cm under *in vivo* conditions. The ultrasound generator is capable of generating ultrasound at a power level of from about 0.05W/cm² to about 100W/cm².

It will be appreciated that the apparatus may be provided in separate units, that is an electric field generator and an ultrasound generator, or in the form of an integrated device.

A preferred embodiment of an apparatus is now described. Such a device comprises an electrode array (which may be a double or multiple needle array with each diagonal representing opposing poles) set around and insulated from an ultrasound delivery device using an insulation ring consisting of a material which absorbs ultrasound and insulate the electrodes from short circuit discharge. The size of the device may vary from a device suitable for treatment of surface lesions to a device which may be inserted into the body using catheterisation or laparoscopy.

Discharge of the electric pulse is accomplished using a suitable electrical source or power pack and ultrasound will be delivered before, during or after delivery of electric pulses.

The electrode array may be configured differently. For example, it may consist of an insulated micro-electrode inserted into the tissue and the opposing electrode array, mounted onto the ultrasonic head inserted into the surrounding tissues. Various electrode arrays are known in the art, and are described in for example, US 5,720,921, WO99/62592, WO 98 56893 A, US 6,041,252 and US 5,873,849. The device as described here may employ any one or more of such electrode configurations.

The electrodes may suitably comprise means for introduction of a substance, preferably a liquid substance, into the tissue. For example, the electrodes may comprise injector needles. Such needles may be used for example to administer a cell death facilitating agent to the tumour cells, or saline to flush out dead cells.

The apparatus is preferably adapted to deliver ultrasound and/or electric fields to a portion of a patient's body, for example an organ. Accordingly, either or both of the ultrasound generator and the electric field generator may comprise a head portion which may be moved so that it is capable of being positioned close to the target to be ablated. Alternatively, a probe may be provided for positioning close to the target, suitably an internal target. For example, if cells in the liver are to be targeted, a ultrasound probe may be positioned on the patient's abdomen so that ultrasound is substantially delivered to the liver. The head portion or probe may be connected by a suitable connection such as a cable to the rest of the device

In another embodiment, the electrosensitisation means is capable of generating an electric field as a stimulus to disrupt an already sensitised cell, for example, an ultrasound sensitised cell. In this embodiment, therefore, the sensitisation means comprises an ultrasound generating means while the disruption means comprises an electric field generating means. The ultrasound generating means therefore is capable of generating ultrasound to sensitise a cell, preferably a nucleated cell, to disruption by a stimulus such as an energy source, e.g., an electric field. Such an embodiment may in general comprise the electrosensitisation means and the ultrasound generating means as set out in the embodiment above, and in particular may function similarly to the device shown in Figure 4.

Each of the applications and patents mentioned above, and each document cited or referenced in each of the foregoing applications and patents, including during the prosecution of each of the foregoing applications and patents ("application cited documents") and any manufacturer's instructions or catalogues for any products cited or mentioned in each of the foregoing applications and patents and in any of the application cited documents, are hereby incorporated herein by reference. Furthermore, all documents cited in this text, and all documents cited or referenced in documents cited in this text, and any manufacturer's instructions or catalogues for any products cited or mentioned in this text, are hereby incorporated herein by reference.

Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. An apparatus for ablation of nucleated cells, the apparatus comprising:
(a) electric field signal generating circuitry for generating an electric field signal;
(b) an electric field delivery component connected to receive the electric field signal and operable to deliver an electric field to a treatment site;
(c) ultrasound signal generating circuitry for generating an ultrasound signal;
(d) an ultrasound delivery component connected to receive the ultrasound signal and operable to deliver ultrasound to the treatment site; and
(e) a controller operable to control (i) the electric field signal generating circuitry such that the electric field delivery component delivers an electric field of a strength operable to sensitise a nucleated cell at the treatment site, and (ii) the ultrasound signal generating circuitry in order to ablate a nucleated cell at the treatment site.

2. A nucleated cell ablation apparatus according to Claim 1, in which the ultrasound is operable to ablate a sensitised nucleated cell at the treatment site.

3. A nucleated cell ablation apparatus according to any preceding claim, in which the electric field delivery component and the ultrasound delivery component are housed in a common delivery head.

4. A nucleated cell ablation apparatus according to any preceding claim, in which the controller is operable to provide the electric field before the ultrasound to the treatment site.

5. A nucleated cell ablation apparatus according to any preceding claim, in which the controller is operable to provide the electric field and the ultrasound to the treatment site simultaneously.

6. A nucleated cell ablation apparatus according to any preceding claim, in which the electric field delivery component comprises an electrical contact.

7. A nucleated cell ablation apparatus according to any preceding claim, in which the electric field delivery component comprises a plurality of electrical contacts positioned at the vertices of a regular polygon, preferably a triangle, a square, a pentagon, a hexagon, or a heptagon.

8. A nucleated cell ablation apparatus according to Claim 7, which further comprises one or more contact electrodes arranged within the circumference of the polygon.

9. A nucleated cell ablation apparatus according to any preceding claim, in which the electric field delivery component comprises a plurality of contact electrodes arranged in a grid.

10. A nucleated cell ablation apparatus according to any of Claims 6 to 9, in which the or each of the contact electrodes comprises a needle.

11. A nucleated cell ablation apparatus according to Claim 10, in which the or each needle is hollow.

12. A nucleated cell ablation apparatus according to Claim 11 in which the apparatus is operable to deliver a cell-death facilitating agent to the vicinity of the nucleated cell via the or each hollow needle.

13. A nucleated cell ablation apparatus according to Claim 12, in which the apparatus further comprises a reservoir containing the cell-death facilitating agent and a means for conveying the cell death facilitating agent through the hollow needle to the vicinity of the nucleated cell.

14. A nucleated cell ablation apparatus according to Claim 10, 11, 12 or 13 in which the or each needle is electrically insulated along at least a portion of its extent, preferably by means of an extendible insulating sleeve.

15. A nucleated cell ablation apparatus according to any preceding claim, in which the electric field delivery component comprises an electrical contact comprising a conductive part of a contact pad.

16. A nucleated cell ablation apparatus according to Claim 15, in which the conductive part of the conductive pad is formed using printed circuit techniques.

17. A nucleated cell ablation apparatus according to Claim 16, in which the apparatus is operable to connect a plurality of electric field signals to a plurality of electrical contacts.

18. A nucleated cell ablation apparatus according to any preceding claim, in which the apparatus is operable to deliver electric fields of between 1 V/cm and 10 kV/cm to the treatment site.

19. A nucleated cell ablation apparatus according to any preceding claim further comprising a diagnostic ultrasound component operable to provide ultrasound images of the treatment site.

20. A nucleated cell ablation apparatus according to any preceding claim, in which the ultrasound delivery component comprises an ultrasound transducer.

21. A nucleated cell ablation apparatus according to Claim 20, in which the apparatus is operable to connect a plurality of ultrasound transducers to a plurality of ultrasound signals.

22. A nucleated cell ablation apparatus according to any preceding claim, in which the ultrasound signal generating circuitry is operable to deliver ultrasound at an average power density of between 0.1 W/cm² and 7 W/cm² to the treatment site.

23. A nucleated cell ablation apparatus according to any preceding claim, in which the ultrasound delivery component is adapted to be coupled to a tissue surface via a contact pressure vessel arranged to distort the tissue surface.

24. A nucleated cell ablation apparatus according to Claim 23, in which the tissue surface is distorted so as to provide focussing of the ultrasound at the treatment site.

25. Use of a nucleated cell ablation apparatus according to any preceding claim in a method of nucleated cell or tissue ablation *in vitro* or *ex vivo.*

26. A method of ablating a nucleated cell *in vitro* or *ex vivo,* the method comprising the steps of:
(a) providing an apparatus according to any of claims 1-24 operable to generate an electric field signal and an ultrasound signal under control of the controller,
(b) generating an electric field signal to cause an electric field delivery component of the apparatus to deliver an electric field to a nucleated cell so as to sensitise it; and
(c) generating an ultrasound signal to cause an ultrasound delivery component of the apparatus to deliver ultrasound to the nucleated cell.

27. A method according to Claim 26, in which the apparatus comprises electric field signal generating circuitry for generating an electric field signal and ultrasound signal generating circuitry for generating an ultrasound signal.

## Patentansprüche

1. Vorrichtung für die Ablation kernhaltiger Zellen, wobei die Vorrichtung folgendes aufweist:
(a) einen elektrische Feldsignale erzeugenden Schaltkreis, um ein elektrisches Feldsignal zu erzeugen,
(b) eine Komponente für die Zuführung von elektrischen Feldern, die so angeschlossen ist, daß sie das elektrische Feldsignal empfängt; und so betreibbar ist, daß sie ein elektrisches Feld einer Behandlungsstelle zuführt,
(c) einen Ultraschallsignale erzeugenden Schaltkreis, um ein Ultraschallsignal zu erzeugen,
(d) eine Ultraschall-Zuführungskomponente, die so angeschlossen ist, daß sie das Ultraschallsignal empfängt, und so betreibbar ist, daß sie der Behandlungsstelle Ultraschall zuführt, und
(e) eine Steuerung, die so betreibbar ist, daß sie (i) den elektrische Feldsignale erzeugenden Schaltkreis so steuert, daß die Komponente für die Zuführung von elektrischen Feldern ein elektrisches Feld mit einer Stärke zuführt, die so betreibbar ist, daß sie eine kernhaltige Zelle an der Behandlungsstelle sensibilisiert, und (ii) den Ultraschallsignale erzeugenden Schaltkreis so steuert, daß eine kernhaltige Zelle an der Behandlungsstelle abladiert bzw. abgetragen wird.

2. Vorrichtung für die Ablation kernhaltiger Zellen nach Anspruch 1, wobei der Ultraschall so betreibbar ist, daß er eine sensibilisierte Zelle an der Behandlungsstelle abladiert.

3. Vorrichtung für die Ablation kernhaltiger Zellen nach einem der vorangegangenen Ansprüche, wobei die Komponente für die Zuführung elektrischer Felder und die Ultraschall-Zuführungskomponente in einem gemeinsamen Zuführungskopf aufgenommen sind.

4. Vorrichtung für die Ablation kernhaltiger Zellen nach einem der vorangegangenen Ansprüche, wobei die Steuerung so betreibbar ist, daß sie der Behandlungsstelle das elektrische Feld vor dem Ultraschall zuführt.

5. Vorrichtung für die Ablation kernhaltiger Zellen nach einem der vorangegangenen Ansprüche, wobei die Steuerung so betreibbar ist, daß sie das elektrische Feld und den Ultraschall der Behandlungsstelle gleichzeitig zuführt.

6. Vorrichtung für die Ablation kernhaltiger Zellen nach einem der vorangegangenen Ansprüche, wobei die Komponente für die Zuführung elektrischer Felder einen elektrischen Kontakt aufweist.

7. Vorrichtung für die Ablation kernhaltiger Zellen nach einem der vorangegangenen Ansprüche, wobei die Komponente für die Zuführung elektrischer Felder eine Mehrzahl von elektrischen Kontakten aufweist, die an den Eckpunkten eines regelmäßigen Polygons, vorzugsweise eines Dreiecks, eines Vierecks, eines Fünfecks, eines Sechsecks oder eines Siebenecks, angeordnet sind.

8. Vorrichtung für die Ablation kernhaltiger Zellen nach Anspruch 7, welche weiterhin eine oder mehrere Kontaktelektroden aufweist, die innerhalb des Umfangs des Polygons angeordnet sind.

9. Vorrichtung für die Ablation kernhaltiger Zellen nach einem der vorangegangenen Ansprüche, wobei die Komponente für die Zuführung elektrischer Felder eine Mehrzahl von in einem Raster angeordneten Kontaktelektroden aufweist.

10. Vorrichtung für die Ablation kernhaltiger Zellen nach einem der Ansprüche 6 bis 9, wobei die oder jede der Kontaktelektroden eine Nadel aufweist.

11. Vorrichtung für die Ablation kernhaltiger Zellen nach Anspruch 10, wobei die oder jede Nadel hohl ist.

12. Vorrichtung für die Ablation kernhaltiger Zellen nach Anspruch 11, wobei die Vorrichtung so betreibbar ist, daß sie in die Nähe der kernhaltigen Zelle ein Zelltod förderndes Mittel über die oder jede hohle Nadel zuführt.

13. Vorrichtung für die Ablation kernhaltiger Zellen nach Anspruch 12, wobei die Vorrichtung weiterhin ein Reservoir, welches das Zelltod fördernde Mittel enthält, und eine Einrichtung zum Befördern des Zelltod fördernden Mittels durch die hohle Nadel in die Nähe der kernhaltigen Zelle aufweist.

14. Vorrichtung für die Ablation kernhaltiger Zellen nach Anspruch 10, 11, 12 oder 13, wobei die oder jede Nadel entlang wenigstens eines Abschnitts ihres Ausmaßes elektrisch isoliert ist, vorzugsweise mittels einer verlängerbaren Isolierhülse.

15. Vorrichtung für die Ablation kernhaltiger Zellen nach einem der vorangegangenen Ansprüche, wobei die Komponente für die Zuführung von elektrischen Feldern einen elektrischen Kontakt aufweist, der einen leitfähigen Teil eines Kontaktfeldes aufweist.

16. Vorrichtung für die Ablation kernhaltiger Zellen nach Anspruch 15, wobei der leitfähige Teil des leitfähigen Feldes unter Verwendung von Leiterplattentechniken gebildet wird.

17. Vorrichtung für die Ablation kernhaltiger Zellen nach Anspruch 16, wobei die Vorrichtung so betreibbar ist, daß sie eine Mehrzahl von elektrischen Feldsignalen mit einer Mehrzahl von elektrischen Kontakten verbindet.

18. Vorrichtung für die Ablation kernhaltiger Zellen nach einem der vorangegangenen Ansprüche, wobei die Vorrichtung so betreibbar ist, daß sie der Behandlungsstelle elektrische Felder von zwischen 1 V/cm und 10 kV/cm zuführt.

19. Vorrichtung für die Ablation kernhaltiger Zellen nach einem der vorangegangenen Ansprüche, welche weiterhin eine diagnostische Ultraschallkomponente aufweist, die so betreibbar ist, daß sie Ultraschallbilder von der Behandlungsstelle bereitstellt.

20. Vorrichtung für die Ablation kernhaltiger Zellen nach einem der vorangegangenen Ansprüche, wobei die Ultraschall-Zuführungskomponente einen Ultraschallwandler aufweist.

21. Vorrichtung für die Ablation kernhaltiger Zellen nach Anspruch 20, wobei die Vorrichtung so betreibbar ist, daß sie eine Mehrzahl von Ultraschallwandlern mit einer Mehrzahl von Ultraschallsignalen verbindet.

22. Vorrichtung für die Ablation kernhaltiger Zellen nach einem der vorangegangenen Ansprüche, wobei der Ultraschallsignale erzeugende Schaltkreis so betreibbar ist, daß er Ultraschall mit einer mittleren Energiedichte von zwischen 0,1 W/cm² und 7 W/cm² an die Behandlungsstelle zuführt.

23. Vorrichtung für die Ablation kernhaltiger Zellen nach einem der vorangegangenen Ansprüche, wobei die Ultraschall-Zuführungskomponente so ausgestaltet ist, daß sie über einen Anpreßdruckbehälter, der so angeordnet ist, daß er die Gewebeoberfläche verzerrt, mit einer Gewebeoberfläche gekoppelt ist.

24. Vorrichtung für die Ablation kernhaltiger Zellen nach Anspruch 23, wobei die Gewebeoberfläche so verzerrt wird, daß eine Fokussierung des Ultraschalls auf die Behandlungsstelle bereitgestellt wird.

25. Verwendung einer Vorrichtung für die Ablation kernhaltiger Zellen nach einem der vorangegangenen Ansprüche in einem Verfahren zur Ablation von kernhaltigen Zellen oder zur Gewebeablation *in vitro* oder *ex vivo.*

26. Verfahren zur Ablation einer kernhaltigen Zelle *in vitro* oder *ex vivo*, wobei das Verfahren die folgenden Schritte umfaßt:
(a) Bereitstellen einer Vorrichtung, die so betreibbar ist, daß sie ein elektrisches Feldsignal und ein Ultraschallsignal unter der Kontrolle durch eine Steuerung erzeugt,
(b) Erzeugen eines elektrischen Feldsignals, um zu bewirken, daß eine Komponente der Vorrichtung für die Zuführung elektrischer Felder einer kernhaltigen Zelle ein elektrisches Feld zuführt, um sie zu sensibilisieren, und
(c) Erzeugen eines Ultraschallsignals, um zu bewirken, daß eine Ultraschall-Zuführungskomponente der Vorrichtung der kernhaltigen Zelle Ultraschall zuführt.

27. Verfahren nach Anspruch 26, wobei die Vorrichtung einen elektrische Feldsignale erzeugenden Schaltkreis aufweist, um ein elektrisches Feldsignal zu erzeugen, und einen Ultraschallsignale erzeugenden Schaltkreis aufweist, um ein Ultraschallsignal zu erzeugen.

## Revendications

1. Dispositif destiné à l'ablation de cellules nucléées, comprenant :
(a) un circuit de génération de signal de champ électrique destiné à générer un signal de champ électrique,
(b) un composant de délivrance de champ électrique connecté pour recevoir le signal de champ électrique et pouvant être mis en oeuvre pour délivrer un champ électrique à un site de traitement,
(c) un circuit de génération de signal ultrasonore destiné à générer un signal ultrasonore,
(d) un composant de délivrance d'ultrasons connecté pour recevoir le signal ultrasonore et pouvant être mis en oeuvre pour délivrer l'ultrason au site de traitement, et
(e) un contrôleur pouvant être mis en oeuvre pour commander (i) le circuit de génération de signal de champ électrique de sorte que le composant de délivrance de champ électrique délivre un champ électrique d'une intensité pouvant être utilisée pour sensibiliser une cellule nucléée au niveau du site de traitement, et (ii) le circuit de génération de signal de champ électrique de manière à effectuer une ablation d'une cellule nucléée au niveau du site de traitement.

2. Dispositif d'ablation de cellules nucléées selon la revendication 1, dans lequel l'ultrason peut être utilisé pour effectuer une ablation d'une cellule nucléée sensibilisée au niveau du site de traitement.

3. Dispositif d'ablation de cellules nucléées selon l'une quelconque des revendications précédentes, dans lequel le composant de délivrance de champ électrique et le composant de délivrance d'ultrasons sont logés dans une tête de délivrance commune.

4. Dispositif d'ablation de cellules nucléées selon l'une quelconque des revendications précédentes, dans lequel le contrôleur peut être mis en oeuvre pour fournir le champ électrique avant l'ultrason au site de traitement.

5. Dispositif d'ablation de cellules nucléées selon l'une quelconque des revendications précédentes, dans lequel le contrôleur peut être mis en oeuvre pour fournir le champ électrique et l'ultrason au site de traitement simultanément.

6. Dispositif d'ablation de cellules nucléées selon l'une quelconque des revendications précédentes, dans lequel le composant de délivrance de champ électrique comprend un contact électrique.

7. Dispositif d'ablation de cellules nucléées selon l'une quelconque des revendications précédentes, dans lequel le composant de délivrance de champ électrique comprend une pluralité de contacts électriques positionnés au niveau des sommets d'un polygone régulier, de préférence un triangle, un carré, un pentagone, un hexagone ou un heptagone.

8. Dispositif d'ablation de cellules nucléées selon la revendication 7, qui comprend en outre une ou plusieurs électrodes de contact disposées sur la circonférence du polygone.

9. Dispositif d'ablation de cellules nucléées selon l'une quelconque des revendications précédentes, dans lequel le composant de délivrance de champ électrique comprend une pluralité d'électrodes de contact disposées dans une grille.

10. Dispositif d'ablation de cellules nucléées selon l'une quelconque des revendications 6 à 9, dans lequel l'électrode de contact ou chacune des électrodes de contact comprend une aiguille.

11. Dispositif d'ablation de cellules nucléées selon la revendication 10, dans lequel l'aiguille ou chaque aiguille est creuse.

12. Dispositif d'ablation de cellules nucléées selon la revendication 11, où le dispositif peut être mis en oeuvre pour délivrer un agent facilitant la mort des cellules à proximité de la cellule nucléée par le biais de l'aiguille creuse ou de chaque aiguille creuse.

13. Dispositif d'ablation de cellules nucléées selon la revendication 12, où le dispositif comprend en outre un réservoir contenant l'agent facilitant la mort des cellules et un moyen destiné à acheminer l'agent facilitant la mort des cellules à travers l'aiguille creuse jusqu'à proximité de la cellule nucléée.

14. Dispositif d'ablation de cellules nucléées selon la revendication 10, 11, 12 ou 13, dans lequel l'aiguille ou chaque aiguille est isolée électriquement le long d'au moins une partie de son étendue, de préférence au moyen d'un manchon d'isolation extensible.

15. Dispositif d'ablation de cellules nucléées selon l'une quelconque des revendications précédentes, dans lequel le composant de délivrance de champ électrique comprend un contact électrique comprenant une partie conductrice d'une plage de connexion de contact.

16. Dispositif d'ablation de cellules nucléées selon la revendication 15, dans lequel la partie conductrice de la plage de connexion conductrice est formée en utilisant des techniques de circuits imprimés.

17. Dispositif d'ablation de cellules nucléées selon la revendication 16, où le dispositif peut être mis en oeuvre pour connecter une pluralité de signaux de champs électriques à une pluralité de contacts électriques.

18. Dispositif d'ablation de cellules nucléées selon l'une quelconque des revendications précédentes, où le dispositif peut être mis en oeuvre pour délivrer des champs électriques entre 1 V/cm et 10 kV/cm au site de traitement.

19. Dispositif d'ablation de cellules nucléées selon l'une quelconque des revendications précédentes, comprenant en outre un composant d'ultrasons de diagnostic pouvant être mis en oeuvre pour fournir des images d'ultrasons du site de traitement.

20. Dispositif d'ablation de cellules nucléées selon l'une quelconque des revendications précédentes, dans lequel le composant de délivrance d'ultrasons comprend un transducteur d'ultrasons.

21. Dispositif d'ablation de cellules nucléées selon la revendication 20, où le dispositif peut être mis en oeuvre pour connecter une pluralité de transducteurs d'ultrasons à une pluralité de signaux ultrasonores.

22. Dispositif d'ablation de cellules nucléées selon l'une quelconque des revendications précédentes, dans lequel le circuit de génération de signal ultrasonore peut être mis en oeuvre pour délivrer un ultrason à une densité de puissance moyenne comprise entre 0,1 W/cm² et 7 W/cm² au site de traitement.

23. Dispositif d'ablation de cellules nucléées selon l'une quelconque des revendications précédentes, dans lequel le composant de délivrance d'ultrasons est conçu pour être relié à une surface de tissu par le biais d'un récipient de pression de contact conçu pour déformer la surface de tissu.

24. Dispositif d'ablation de cellules nucléées selon la revendication 23, dans lequel la surface de tissu est déformée de manière à permettre la concentration de l'ultrason au niveau du site de traitement.

25. Utilisation d'un dispositif d'ablation de cellules nucléées selon l'une quelconque des revendications précédentes dans un procédé d'ablation de cellules nucléées ou de tissu *in vitro* ou *ex vivo.*

26. Procédé d'ablation d'une cellule nucléée *in vitro* ou *ex vivo,* le procédé comprenant les étapes consistant à :
(a) fournir un dispositif pouvant être mis en oeuvre pour générer un signal de champ électrique et un signal ultrasonore sous la commande d'un contrôleur,
(b) générer un signal de champ électrique pour amener un composant de délivrance de champ électrique du dispositif à délivrer un champ électrique à une cellule nucléée de manière à la sensibiliser, et
(c) générer un signal ultrasonore pour amener un composant de délivrance d'ultrasons du dispositif à délivrer l'ultrason à la cellule nucléée.

27. Procédé selon la revendication 26, dans lequel le dispositif comprend le circuit de génération de signal de champ électrique destiné à générer un signal de champ électrique et un circuit de génération de signal ultrasonore destiné à générer un signal ultrasonore.
